# EUROPEAN PATENT APPLICATION

(11) **EP 4 209 588 A1**
(43) Date of publication of application: **12.07.2023**
(21) Application number: 21864429.2
(22) Date of filing: 03.09.2021
(51) Int. Cl.: C12N 15/09

(54) **METHOD FOR EDITING TARGET DNA, METHOD FOR PRODUCING CELL HAVING EDITED TARGET DNA, AND DNA EDITION SYSTEM FOR USE IN SAID METHODS**

(30) Priority: 04.09.2020 JP 2020148944
(71) Applicant: Hiroshima University, Higashi-Hiroshima-shi Hiroshima 739-8511 (JP); SUMITOMO CHEMICAL COMPANY, LIMITED, Tokyo 103-6020 (JP)
(72) Inventor: SAKUMA, Tetsushi, Hiroshima-shi, Hiroshima 739-8511 (JP); YAMAMOTO, Takashi, Hiroshima-shi, Hiroshima 739-8511 (JP); NISHIBORI, Nahoko, Hiroshima-shi, Hiroshima 739-8511 (JP); YOSHIMA, Tadahiko, Osaka-shi, Osaka 554-8558 (JP); HORIGOME, Kazuhiko, Osaka-shi, Osaka 554-8558 (JP)
(74) Representative: J A Kemp LLP
(86) International application number: PCT/JP2021/032436
(87) International publication number: WO 2022/050377

(57) **Abstract**

A method for editing a target DNA comprising:
a step of bringing
(1) a fusion protein containing a TALE and a nucleic acid base converting enzyme and
(2) a CRISPR-Cas9 system containing a Cas9 protein which has lost part or all of a nuclease activity and a guide RNA thereof

into contact with a target DNA to edit a base in a target site of the target DNA by using a nucleic acid base converting enzyme activity of the fusion protein, wherein
a TALE recognition sequence recognized by the TALE in the fusion protein or a complementary sequence thereof is present on a 5' side of the target site via a spacer 1 having a chain length of 7 to 31 bp, and
a guide RNA-target sequence recognized by the guide RNA in the CRISPR-Cas9 system is present to contain a complementary base of the target site.

## Description

### [Technical Field]

The present invention relates to a method for editing a target DNA, a method for producing a cell in which a target DNA is edited, and a DNA editing system for use in these, and particularly relates to a method for editing a target DNA, a method for producing a cell genome-edited by using the above method, and a combination for use in these.

### [Background Art]

Since the first report (Komor, A. et al., Nature 533, 420-424 (2016), DOI: 10.1038/nature 17946 (NPL 1)) was made in 2016, base-editing methods in which CRISPR-dCas9/nCas9 and nucleic acid base converting enzymes such as deaminases are combined have rapidly spread because of their high editing efficiency and simplicity with no need of donor DNAs, examples of implementing the methods in various living organisms and cells have been reported, and many improved systems have also been developed (Rees, H. A. and Liu, D. R., Nat Rev Genet 19, 770-788 (2018), DOI: 10.1038/s41576-018-0059-1 (NPL 2) and the like).

For example, in base editing from base C to T (C_{→}T (G_{→}A)), a system utilizing APOBEC1 of rats (rAPOBEC1) (from BE3 in the above-described firstly published paper, improvement to a system called AncBE4max has been currently proceeded; Koblan, L. et al., Nat Biotechnol 36, 843-846 (2018), DOI: 10.1038/nbt.4172 (NPL 3)), a system utilizing CDA1 of lampreys (PmCDA1) (Target-AID; Nishida, K. et al., Science 353, aaf8729 (2016), DOI: 10.1126/science.aaf8729 (NPL 4)), and the like are often used, and it has been also reported that APOBEC3A, APOBEC3B, and the like can be utilized (Gehrke, J. et al., Nat Biotechnol 36, 977-982 (2018), DOI: 10.1038/nbt.4199 (NPL 5); Martin, A. S. et al., Sci Rep 9, 497 (2019), DOI: 10.1038/s41598-018-36739-9 (NPL 6)). In addition, for example, as a base editing system from base A to G (A_{→}G (T_{→}C)), an Adenine Base Editor (ABE) system that is constructed by engineering TadA protein derived from *Escherichia* coli has also been reported (Gaudelli, N. et al., Nature 551, 464-471 (2017), DOI: 10.1038/nature24644 (NPL 7)).

However, in all of these systems, the specificity of target genome regions is dependent only on the PAM sequences recognized by guide RNAs and dCas9/nCas9, which are the components of CRISPR-dCas9/nCas9. Meanwhile, a system in which deaminases are fused to the zinc finger (ZF) or the TAL effector (TALE) has also been reported (Yang, L. et al., Nat Commun 7, 13330 (2016), DOI: 10.1038/ncomms13330 (NPL 8)). However, DNA-binding proteins such as ZF and TALE do not have a property to separate double-stranded DNAs into single-stranded DNAs unlike CRISPR-dCas9/nCas9, the efficiency of the base editing with deaminases using single-stranded DNAs as substrates is significantly low.

Under such circumstances, a system in which deaminases are split into a N-terminal domain and a C-terminal domain, which are then fused to ZF and nCas9, respectively, so that base editing is only applied when target sequences of both ZF and nCas9 are present in the vicinity has been reported (International Publication No. WO2018/218166). It is considered that using such a system makes it possible to improve the specificity for the addition of the recognition sequence of ZF as compared with the conventional CRISPR-dCas9/nCas9-dependent systems.

### [Citation List]

### [Patent Literature]

[PTL 1] International Publication No. WO2018/218166

### [Non Patent Literature]

[NPL 1] Komor, A. et al., Nature 533, 420-424 (2016)
[NPL 2] Rees, H. A. and Liu, D. R., Nat Rev Genet 19, 770-788 (2018)
[NPL 3] Koblan, L. et al., Nat Biotechnol 36, 843-846 (2018)
[NPL 4] Nishida, K. et al., Science 353, aaf8729 (2016)
[NPL 5] Gehrke, J. et al., Nat Biotechnol 36, 977-982 (2018)
[NPL 6] Martin, A. S. et al., Sci Rep 9, 497 (2019)
[NPL 7] Gaudelli, N. et al., Nature 551, 464-471 (2017)
[NPL 8] Yang, L. et al., Nat Commun 7, 13330 (2016)

### [Summary of Invention]

### [Technical Problem]

However, the efficiency of the base editing using the system described in PTL 1 is, even at most, around 1/3 of the BE3 (NPL 1), which is a conventional base editing system, and it has been considered that this is caused due to a decrease in activity which is caused by splitting.

The present invention has been made in view of the above-described problems of the conventional techniques, and an object thereof is to provide a method capable of specifically and efficiently editing a target DNA by using a nucleic acid base converting enzyme, a method for producing a cell genome-edited by using the above method, and a DNA editing system for use in these.

### [Solution to Problem]

As a result of conducting earnest studies in order to achieve the above-described object, the present inventors have developed a system that fuses a nucleic acid base converting enzyme to TALE without splitting, and binds both of a TALE-nucleic acid base converting enzyme fusion protein (preferably, TALE-deaminase-UGI fusion protein) and a dCas9/guide RNA complex or nCas9/guide RNA complex in the vicinity of a target site of a target DNA. The structure which does not split a nucleic acid base converting enzyme is in compliance with AncBE4max, which is an already reported highly active system in the case of deaminases, for example. However, since this is not directly linked to dCas9 or nCas9 but is linked to TALE, even when only the TALE-nucleic acid base converting enzyme (deaminase) fusion protein binds to the target DNA, base editing hardly occurs because the single-stranded DNA, which serves as a substrate, is not exposed. In addition, even when only the dCas9/guide RNA complex or only the nCas9/guide RNA complex binds to the target DNA, base editing again does not occur because the nucleic acid base converting enzyme is not present. Hence, the present inventors have found that base editing occurs in the first place when both of the TALE-nucleic acid base converting enzyme fusion protein and the dCas9/guide RNA complex or nCas9/guide RNA complex bind to the target DNA with a specific distance therebetween about the target site, so that high specificity was secured, and high editing efficiency can be achieved because the nucleic acid base converting enzyme is not split.

In addition, the present inventors have verified that in a combination of the above CRISPR-dCas9 or nCas9 and the TALE-nucleic acid base converting enzyme fusion protein, base editing efficiency similar to that of the conventional AncBE4max system (the maximum activity with reporter assay) can be achieved by studying the spacer 1 between the TALE recognition sequence (or a complementary sequence thereof) and the target site on the target DNA and the position of the RNA target sequence on the target DNA, and have thus completed the present invention. Aspects of the present invention achieved based on the above-described findings are as follows:
[1] A method for editing a target DNA comprising:
   a step of bringing
      (1) a fusion protein containing a TALE and a nucleic acid base converting enzyme and
      (2) a CRISPR-Cas9 system containing a Cas9 protein which has lost part or all of a nuclease activity and a guide RNA thereof
   into contact with a target DNA to edit a base of a target site of the target DNA by using a nucleic acid base converting enzyme activity of the fusion protein, wherein
   a TALE recognition sequence recognized by the TALE in the fusion protein or a complementary sequence thereof is present on a 5' side of the target site via a spacer 1 having a chain length of 7 to 31 bp, and
   a guide RNA-target sequence recognized by the guide RNA in the CRISPR-Cas9 system is present to contain a complementary base of the target site.
[2] A method for producing a cell in which a target DNA is edited, comprising:
   a step of introducing or expressing
      (1) a fusion protein containing a TALE and a nucleic acid base converting enzyme and
      (2) a CRISPR-Cas9 system containing a Cas9 protein which has lost part or all of a nuclease activity and a guide RNA thereof
   into a cell or in a cell to bring the fusion protein and the CRISPR-Cas9 system into contact with a target DNA to edit a base in a target site of the target DNA by using a nucleic acid base converting enzyme activity of the fusion protein, wherein
   a TALE recognition sequence recognized by the TALE in the fusion protein or a complementary sequence thereof is present on a 5' side of the target site via a spacer 1 having a chain length of 7 to 31 bp, and
   a guide RNA-target sequence recognized by the guide RNA in the CRISPR-Cas9 system is present to contain a complementary base of the target site.
[3] The method according to [1] or [2], wherein
   the fusion protein further contains a linker 1 which binds the TALE and the nucleic acid base converting enzyme.
[4] The method according to any one of [1] to [3], wherein
   the fusion protein further contains a base excision repair inhibitor which is bound to a C-terminal side via a linker 2.
[5] The method according to any one of [1] to [4], wherein
   the nucleic acid base converting enzyme in the fusion protein is a deaminase.
[6] The method according to [5], wherein
   the deaminase is at least one selected from the group consisting of APOBEC, PmCDA1, Anc689, and TadA.
[7] A DNA editing system for use in the method according to any one of [1] to [6], comprising:
   (1) a fusion protein containing a TALE and a nucleic acid base converting enzyme; and
   (2) a CRISPR-Cas9 system containing a Cas9 protein which has lost part or all of a nuclease activity and a guide RNA thereof.
[8] The DNA editing system according to [7], wherein
   the fusion protein further contains a linker 1 which binds the TALE and the nucleic acid base converting enzyme.
[9] The DNA editing system according to [7] or [8], wherein
   the fusion protein further contains a base excision repair inhibitor which is bound to a C-terminal side via a linker 2.
[10] The method according to any one of [7] to [9], wherein
   the nucleic acid base converting enzyme in the fusion protein is a deaminase.
[11] The DNA editing system according to [10], wherein
   the deaminase is at least one selected from the group consisting of APOBEC, PmCDA1, Anc689, and TadA.

### [Advantageous Effects of Invention]

The present invention makes it possible to provide a method capable of specifically and efficiently editing a target DNA by using a nucleic acid base converting enzyme, a method for producing a cell genome-edited by using the method, and a DNA editing system for use in these.

### [Brief Description of Drawings]

[Fig. 1] Fig. 1 is a concept diagram showing the structure of a reporter plasmid.
[Fig. 2] Fig. 2 is a concept diagram showing the structure of the sequence from a NheI recognition site to an XhoI recognition site in the reporter plasmid.
[Fig. 3] Fig. 3 is a schematic diagram of the structure of a NanoLuc expressing reporter and one aspect of base editing by using a TALE-deaminase.
[Fig. 4] Fig. 4 is a concept diagram showing one aspect of the structure of the TALE-deaminase.
[Fig. 5] Fig. 5 is a graph showing AncBE4max activity values obtained in Test 1.
[Fig. 6] Fig. 6 is a graph showing TALE-deaminase activity values (proportions to AncBE4max activity values) obtained in Test 2 ((A) WT-104-BE4, (B) 47-104-BE4).
[Fig. 7] Fig. 7 is a graph showing TALE-deaminase activity values (proportions to AncBE4max activity values) obtained in Test 2 ((A) WT-104-AID, (B) 63-104-AID, (C) 47-104-AID, (D) WT-12-AID, (E) 63-12-AID, (F) 47-12-AID).
[Fig. 8] Fig. 8 is a graph showing TALE-deaminase activity values (proportions to AncBE4max activity values) obtained in Test 3 ((A) WT-104-AID, (B) 63-104-AID, (C) 47-104-AID, (D) WT-12-AID, (E) 63-12-AID, (F) 47-12-AID).
[Fig. 9] Fig. 9 is a graph showing relation between TALE-deaminase activity values (proportions to AncBE4max activity values) obtained in Test 4 and the length of the linker 1.
[Fig. 10] Fig. 10 is a schematic diagram of the structure of the NanoLuc expressing reporter and one aspect of base editing by using the TALE-deaminase.
[Fig. 11] Fig. 11 is concept diagrams showing one aspects (A, B) of the structures of the TALE-deaminases.
[Fig. 12A] Fig. 12A is a graph showing TALE-deaminase activity values (proportions to AncBE4max activity values) obtained in Test 5 ((A) BE4-TALE-WT: BE4-32-WT, BE4-135-WT, and BE4-234-WT, (B) BE4-TALE-63: BE4-32-63, BE4-135-63, BE4-234-63).
[Fig. 12B] Fig. 12B is a graph showing TALE-deaminase activity values (proportions to AncBE4max activity values or ABE8e activity values) obtained in Test 5 ((C) BE4-TALE-47: BE4-32-47, BE4-135-47, and BE4-234-47, (D) ABE8e-TALE-47: ABE8e-32-47, ABE8e-135-47, AB E 8 e - 2 3 4 - 4 7) .
[Fig. 13A] Fig. 13A is concept diagrams showing target regions (AS3 (a), AS14 (b), S2 (c)) on endogenous DNAs (CCR5, HBB), the positions of the TALE recognition sequences, and the positions of complementary sequences of the target sequences of the guide RNA.
[Fig. 13B] Fig. 13B is concept diagrams showing target regions (AS5 (d), AS14 (e), AS6 (f), AS7 (g)) on endogenous DNAs (CCR5, HBB), the positions of the TALE recognition sequences and complementary sequences thereof, and the positions of complementary sequences of target sequences of the guide RNA.
[Fig. 14] Fig. 14 is a graph showing proportions of T at the 1st base (A), the 10th base (B), and the 11th base (C) in the complementary sequences of the target sequences of the guide RNA (sgRNA-AS3), on the target AS3 when TALE-AID was used, which were obtained in Test 6.
[Fig. 15] Fig. 15 is a graph showing proportions of T at the 1st base (A), the 5th base (B), and the 6th base (C) in the complementary sequences of the target sequences of the guide RNA (sgRNA-AS14), on the target AS14 when TALE-AID was used, which were obtained in Test 6.
[Fig. 16] Fig. 16 is a graph showing proportions of T at the 1st base (A), the 8th base (B), and the 9th base (C) in the complementary sequences of the target sequences of the guide RNA (sgRNA-S2), on the target S2 when TALE-AID was used, which were obtained in Test 6.
[Fig. 17] Fig. 17 is a graph showing proportions of T at the 7th base (A) and the 8th base (B) in the complementary sequences of the target sequences of the guide RNA (sgRNA-AS5), on the target AS5 when BE4-TALE was used, which were obtained in Test 6.
[Fig. 18] Fig. 18 is a graph showing proportions of T at the 1st base (A), the 5th base (B), and the 6th base (C) in the complementary sequences of the target sequences of the guide RNA (sgRNA-AS14), on the target AS14 when BE4-TALE was used, which were obtained in Test 6.
[Fig. 19] Fig. 19 is a graph showing proportions of T at the 5th base (A) and the 6th base (B) in the complementary sequences of the target sequences of the guide RNA (sgRNA-AS6), on the target AS6 when BE4-TALE was used, which were obtained in Test 6.
[Fig. 20] Fig. 20 is a graph showing proportions of T at the 6th base (A) and the 7th base (B) in the complementary sequences of the target sequences of the guide RNA (sgRNA-AS7), on the target AS7 when BE4-TALE was used, which were obtained in Test 6.
[Fig. 21] Fig. 21 is a graph showing proportions of G at the 3rd base (A) and the 4th base (B) in the complementary sequences of the target sequences of the guide RNA (sgRNA-AS5), on the target AS5 when ABE8e-TALE was used, which were obtained in Test 6.
[Fig. 22] Fig. 22 is a graph showing proportions of G at the 4th base (A) and the 7th base (B) in the complementary sequences of the target sequences of the guide RNA (sgRNA-AS6), on the target AS6 when ABE8e-TALE was used, which were obtained in Test 6.
[Fig. 23] Fig. 23 is a graph showing proportions of G at the 4th base (A) and the 8th base (B) in the complementary sequences of the target sequences of the guide RNA (sgRNA-AS7), on the target AS7 when ABE8e-TALE was used, which were obtained in Test 6.

### [Description of Embodiments]

Hereinafter, the present invention is described in more detail by giving a preferred embodiment as an example; however, the present invention is not limited to this.

### <Target DNA editing method>

The target DNA editing method of the present invention is a method for editing a target DNA, comprising:
a step of bringing
   (1) a fusion protein containing a TALE and a nucleic acid base converting enzyme and
   (2) a CRISPR-Cas9 system containing a Cas9 protein which has lost part or all of a nuclease activity and a guide RNA thereof
into contact with a target DNA to edit a base in a target site of the target DNA by using a nucleic acid base converting enzyme activity of the fusion protein, wherein
a TALE recognition sequence recognized by the TALE in the fusion protein or a complementary sequence thereof is present on a 5' side of the target site via a spacer 1 having a chain length of 7 to 31 bp, and
a guide RNA-target sequence recognized by the guide RNA in the CRISPR-Cas9 system is present to contain a complementary base of the target site.

### (Fusion protein)

The fusion protein according to the present invention is a fusion protein containing a TALE and a nucleic acid base converting enzyme. In the Specification, the fusion protein according to the present invention is sometimes referred to as a "TALE-nucleic acid base converting enzyme fusion protein" or a "TALE-nucleic acid base converting enzyme". As the fusion protein, the TALE and the nucleic acid base converting enzyme may be bound in this order, or the nucleic acid base converting enzyme and the TALE may be bound in this order, from the N-terminus. In addition, the fusion protein according to the present invention may contain two or more (preferably, two) nucleic acid base converting enzymes, and in this case, the nucleic acid base converting enzymes may be of one type or may be a combination of two or more types. For example, in the case where the fusion protein according to the present invention contains two nucleic acid base converting enzymes (a first nucleic acid base converting enzyme and a second nucleic acid base converting enzyme), these may be bound in the order of the first nucleic acid base converting enzyme, the TALE, and the second nucleic acid base converting enzyme. Among these, the fusion protein according to the present invention is preferably such that the TALE and the nucleic acid base converting enzyme are bound in this order from the N-terminus or the nucleic acid base converting enzyme and the TALE are bound in this order from the N-terminus.

### [TALE]

The TALE (Transcription Activator-Like Effector) is a protein which is secreted by proteobacteria in Xanthomonas and activates the gene transcription of the host plant. The "TALE" according to the present invention contains at least an N-terminal domain and a TALE repeat domain, and may further contain a C-terminal domain.

The TALE repeat domain is composed of multiple, for example 10 to 30, preferably 13 to 25, and more preferably 15 to 20 tandem repeats (TALE repeats) of TALE sequences which form a right-handed superhelix (superhelical). A one unit of typical TALE repeats (one TALE sequence) is composed of 33 to 35 amino acids, and recognizes a specific base of DNA by using a variable residue (repeat variable di-residue: RVD) composed of 12th and 13th, two amino acid residues. Examples of RVDs that specifically recognize bases include HD which recognizes C, NG which recognizes T, NI which recognizes A, NN which recognizes G or A, NS which recognizes A, C, G, or T, and the like. It is possible to create a TALE which can recognize and bind to a desired base sequence (TALE recognition sequence) on DNA by artificially linking a TALE sequence which recognizes a specific base based on the DNA recognition mechanism of the TALE repeat domain.

The TALE sequence of the TALE according to the present invention may be those obtained by appropriately modifying naturally occurring amino acid sequences as long as the TALE repeat domains of these each can recognize and bind to TALE recognition sequences. For example, in the TALE sequence, the positions of RVDs may each independently be changed, or one or multiple (for example, 10 or less, 9 or less, 8 or less, 7 or less, 6 or less, preferably 5 or less, or 4 or less, further preferably 3 or less or 2 or less) amino acid residues other than the RVDs may be substituted, inserted, and/or deleted. In addition, the 12th and 13th, two amino acid residues of the RVDs may each be substituted with another amino acid residue in order to enhance the specificity to A, T, C, and G.

In addition, the TALE repeat domain of the TALE according to the present invention is designed to recognize a base sequence or a complementary sequence thereof present on a 5' side of the target site of the target DNA via a spacer 1 having a chain length of 7 to 31 bp, that is, designed such that a TALE recognition sequence recognized by the TALE or a complementary sequence thereof is a base sequence present on the 5' side of the target site via a spacer 1 having a chain length of 7 to 31 bp. Techniques for designing and preparing such desired TALEs are known (for example, Miller et al., Nat. Biotechnol., 29, 143-148 (2011); Sakuma et al., Sci. Rep., 3: 3379 (2013)), and a TALE having a high binding activity to the base sequence can be prepared by using a Platinum Gate system described in Sakuma et al. (2013), for example.

As the N-terminal domain of the TALE according to the present invention, a naturally occurring amino acid sequence (for example, an amino acid sequence of an N-terminal domain contained in pTALETF_v2 (ID: 32185 to 32188) of Addgene) can be used; however, one obtained by appropriately modifying the naturally occurring amino acid sequence may be used as long as it does not exert adverse effects on the functions (the binding capability to the TALE recognition sequence, the editing capability in the target site, and the like) of the fusion protein according to the present invention. For example, one or multiple (for example, 50 or less, 30 or less, 20 or less, 10 or less, 9 or less, 8 or less, 7 or less, 6 or less, preferably 5 or less or 4 or less, further preferably 3 or less or 2 or less) amino acid residues may be substituted, inserted, and/or deleted. In addition, a FLAG-tag for purification and detection, a nuclear localization signal (NLS) for transporting the TALE-nucleic acid base converting enzyme into cell nuclei, and the like may be contained.

As such N-terminal domain, the chain length of the N-terminal domain is preferably 49 to 287 amino acid residues, more preferably 80 to 200 amino acid residues, and further preferably 120 to 180 amino acid residues. If the chain length of the N-terminal domain is less than the above-described lower limit, the binding to the target DNA tends to be failed, while if the chain length of the N-terminal domain is more than the above-described upper limit, the expression efficiency in the case of expressing a fusion protein tends to decrease.

Specific examples of the N-terminal domain of the TALE according to the present invention include, for example, an amino acid sequence of an N-terminal domain contained in ptCMV-136/63-VR-HD (ID: 50699) of Addgene and an amino acid sequence of an N-terminal domain contained in ptCMV-153/47-VR-HD (ID: 50703) of Addgene, but the N-terminal domain is not limited to these.

In the case where the TALE according to the present invention further contains a C-terminal domain, as the C-terminal domain of the TALE, a naturally occurring amino acid sequence (for example, an amino acid sequence of a C-terminal domain contained in pTALETF_v2 (ID: 32185 to 32188) of Addgene) can be used; however, one obtained by appropriately modifying the naturally occurring amino acid sequence may be used as long as it does not exert adverse effects on the functions (the binding capability to the TALE recognition sequence, the editing capability in the target site, and the like) of the fusion protein according to the present invention. For example, one or multiple (for example, 50 or less, 30 or less, 20 or less, 10 or less, 9 or less, 8 or less, 7 or less, 6 or less, preferably 5 or less or 4 or less, further preferably 3 or less or 2 or less) amino acid residues may be substituted, inserted, and/or deleted.

In the case where the TALE according to the present invention further contains a C-terminal domain, the C-terminal domain of the TALE may be a naturally occurring amino acid sequence in which some amino acid sequences on the C-terminal side are removed. As such C-terminal domain, the chain length of the C-terminal domain is preferably 1 to 180 amino acid residues, more preferably 20 to 100 amino acid residues, and further preferably 40 to 70 amino acid residues. If the chain length of the C-terminal domain is more than the above-described upper limit, the expression efficiency in the case of expressing a fusion protein tends to decrease.

Specific examples of the C-terminal domain include, for example, an amino acid sequence of a C-terminal domain contained in pTALETF_v2 (ID: 32185 to 32188) of Addgene (WT: the number of amino acids=180), an amino acid sequence of a C-terminal domain contained in pTALEN_v2 (ID: 32189 to 32192) of Addgene (the number of amino acids=63), and an amino acid sequence of a C-terminal domain contained in ptCMV-153/47-VR-NG (ID: 50704) of Addgene (the number of amino acids=47), but the C-terminal domain is not limited to these.

### [Nucleic acid base converting enzyme]

In the present invention, the nucleic acid base converting enzyme means an enzyme capable of converting a target nucleotide to another nucleotide or omitting the target nucleotide without cleaving the DNA strand by catalyzing a reaction for converting a substituent on a purine or pyrimidine ring on a DNA base to another group or atom or omitting the substituent.

Such nucleic acid base converting enzyme is not particularly limited as long as it is capable of catalyzing the above-described reaction, and includes, for example, deaminases and glycosylases, and may be only one of these or two or more of these in combination. Among these, a deaminase is preferable as the nucleic acid base converting enzyme according to the present invention.

### (Deaminase)

The deaminase is an enzyme which catalyzes deamination reaction of converting an amino group of a base to a carbonyl group, and belongs to the nucleic acid/nucleotide deaminase super family. Such deaminase includes cytidine deaminases which can convert cytosine or 5-methylcytosine to uracil or thymine, respectively, adenosine deaminases which can convert adenine to hypoxanthine, and guanosine deaminases which can convert guanine to xanthine, and a desired deaminase can be used depending on the target base substitution.

The source of the deaminase is not particularly limited, and includes, for example, lampreys; and mammals such as human, monkey, pig, cattle, horse, rat, and mouse.

As such deaminase, cytidine deaminases include, for example, APOBEC (rAPOBEC1 derived from rat, hAPOBEC1, hAPOBEC2, hAPOBEC3 (hAPOBEC3A, 3B, 3C, 3D (3E), 3F, 3G, 3H), and hAPOBEC4 derived from human, and the like) ; Anc689, which is an ancestral amino acid sequence of APOBEC; AID (activation-induced cytidine deaminase (AICDA), which is derived from mammals (examples: human, pig, cattle, horse, monkey, and the like)); and PmCDA1 (Petromyzon marinus cytosine deaminase 1), which is in the family of AID and derived from lampreys. In addition, adenosine deaminases include, for example, TadA, which is derived from E. coli. Each of the above-described deaminases includes modifications thereof (for example, TadA-8e, TadA7.10, and further, modifications of these, and the like) . Among these, the deaminase is preferably at least one selected from the group consisting of APOBEC, PmCDA1, Anc689, and TadA (including modifications of these).

In addition, in the case where the fusion protein according to the present invention contains two or more deaminases, the combination of these can be selected as appropriate depending on the target base substitution, and includes, for example, a combination of the same or different cytidine deaminases, a combination of the same or different adenosine deaminases, the same or different guanosine deaminases, a combination of a cytidine deaminase and an adenosine deaminase, a combination of a cytidine deaminase and a guanosine deaminase, and a combination of an adenosine deaminase and a guanosine deaminase. Among these, the combination of deaminases is preferably a combination of TadA and PmCDA1. Note that base sequences and amino acid sequences of these deaminases are known, and can be acquired from a known public database (Genbank or the like).

In addition, the deaminase may be one with modification (for example, substitution, insertion, and/or deletion of the amino acid residue) made based on the base sequences and amino acid sequences of these known deaminases as long as it has the deamination activity (deaminase activity). The presence or absence of the deaminase activity can be checked by using a known method as appropriate. For example, in the case of a cytidine deaminase, the presence or absence of the deaminase activity can be checked by conducting an enzyme reaction using cytidine as a substrate to detect and quantify uridine, which is a metabolite of cytidine.

### [Linker 1]

It is preferable that the fusion protein according to the present invention further contain a linker 1. In this case, in the fusion protein according to the present invention, TALE and the nucleic acid base converting enzyme are linked by the linker 1. In addition, in the case where the fusion protein according to the present invention contains multiple nucleic acid base converting enzymes, it is preferable that multiple linkers 1 be correspondingly present. In the case where the fusion protein according to the present invention contain multiple linkers 1, these may be of only one type, or may be of two or more types in combination.

In the case where the fusion protein according to the present invention further contains a linker 1, the chain length of the linker 1 is not particularly limited, but is preferably 1 to 450 amino acid residues, more preferably 5 to 250 amino acid residues, more preferably 5 to 200 amino acid residues, more preferably 12 to 150 amino acid residues, and further preferably 12 to 104 amino acid residues. If the chain length of the linker 1 is more than the upper limit, the expression efficiency in the case of expressing a fusion protein tends to decrease.

The linker 1 according to the present invention includes, for example, 12 amino acids described in Yang L et al., Nat Commun 7 13330 (2016), 104 amino acids described in Nishida, K. et al., Science 353, aaf8729 (2016), 32 amino acids described in Koblan, L. et al., Nat Biotechnol 36, p. 843-846 (2018), HTS95 amino acid sequence (95 amino acids) described in Sun, N. and Zhao, H., Mol BioSyst 10, p. 446-453 (2014), Doi: 10.1039/c3mb70412b) and repeats of these, but is not limited to these.

In addition, as the fusion protein of the present invention, in the case where the nucleic acid base converting enzyme is located on the C-terminal side of TALE, it is preferable that the TALE repeat domain and the nucleic acid base converting enzyme be separated away by 1 to 630 amino acid residues. For this reason, it is preferable that the fusion protein of the present invention contain at least one of C-terminal domains of the TALE and linkers 1. The chain length between the TALE repeat domain and the nucleic acid base converting enzyme in this case (the number of amino acid residues up to the residue adjacent to the N-terminal side of the N terminus of the nucleic acid base converting enzyme counted as the amino acid residue adjacent to the C-terminal side of the C terminus of the TALE repeat domain is deemed as the 1st residue) is more preferably 25 to 300 amino acid residues, and further preferably 52 to 174 amino acid residues. If the chain length is less than the above-described lower limit, the efficiency of base editing tends to decrease, while if the chain length is more than the above-described upper limit, the expression efficiency in the case of expressing a fusion protein tends to decrease.

Note that the residue at the C terminus of the TALE repeat domain may be the residue at the C terminus of the TALE sequence (preferably 1 unit: 33 to 35 amino acids) located immediately before (on N-terminal side of) a truncated last half-repeat (LHR) located on the C-terminal side of the TALE repeat domain (in the case where there is no LHR, the residue at the C terminus of the last TALE sequence (preferably 1 unit: 33 to 35 amino acids) contained in the TALE repeat domain). In addition, the residue at the N terminus of the nucleic acid base converting enzyme may be the initial residue (normally methionine) of the amino acid sequence having an identity of 85% or more (preferably 90% or more) to the amino acid sequence of a typical nucleic acid base converting enzyme which can be acquired from a known database (for example, NCBI) in accordance with a homology search using a known method (for example, BLAST (NCBI)).

### [Base excision repair inhibitor, linker 2]

It is preferable that the fusion protein according to the present invention further contain a base excision repair inhibitor bound via a linker 2 on the C-terminal side, that is, at the C-terminus of the nucleic acid base converting enzyme (in the case where the nucleic acid base converting enzyme is located on the C-terminal side of the TALE) or the C-terminus of the TALE (in the case where the nucleic acid base converting enzyme is located only on the N-terminal side of the TALE) . In the case where the fusion protein further contains a base excision repair inhibitor, the number of the base excision repair inhibitor may be one or two or more (preferably two) via the linker 2 per fusion protein. In addition, in the case where there are two or more base excision repair inhibitors, the base excision repair inhibitors may be of only one type or may be of two or more types in combination, but are preferably of one type.

The base excision repair inhibitors according to the present invention are not particularly limited as long as they inhibit base excision repair, but DNA glycosylase inhibitors are preferable. The DNA glycosylase inhibitors include thymine-DNA glycosylase inhibitors, uracil-DNA glycosylase inhibitors, oxoguanine-DNA glycosylase inhibitors, and alkylguanine-DNA glycosylase inhibitors. For example, in the case of using a cytidine deaminase, which is a deaminase, as the nucleic acid base converting enzyme, it is appropriate to use the uracil-DNA glycosylase inhibitor (UGI) for inhibiting repair of U:G or G:U mismatch of DNA caused by mutation. In this case, when the deaminase and UGI are present on a single polypeptide, the efficiency of base editing is further improved.

Such an uracil-DNA glycosylase inhibitor includes, for example, an uracil-DNA glycosylase inhibitor (UGI) derived from PBS-1, which is Bacillus subtilis bacteriophage, or an uracil-DNA glycosylase inhibitor (UGI) derived from PBS-2, which is Bacillus subtilis bacteriophage, but is not limited to these. The base sequences and amino acid sequences of these are known and can be acquired from a public database (Genbank or the like). In addition, the base excision repair inhibitor according to the present invention may be one with modification (for example, in which substitution, insertion, and/or deletion of amino acid residues is introduced) made based on the base sequence and amino acid sequence of a known base excision repair inhibitor as long as it has repair inhibitory activity on the above-described mismatches of DNA.

In the case where the fusion protein according to the present invention further contains the base excision repair inhibitors and there are multiple base excision repair inhibitors, the base excision repair inhibitors can be linked via the linkers 2 in such a manner of the fusion protein-the linker 2-the first base excision repair inhibitor-the linker 2-the second base excision repair inhibitor.... The multiple linkers 2 in this case may be of only one type or of two or more types in combination, but are preferably of one type.

Such a linker 2 is not particularly limited, but the chain length of the linker 2 is preferably 1 to 50 amino acid residues, more preferably 5 to 30 amino acid residues, and further preferably 8 to 15 amino acid residues. If the chain length of the linker 2 is less than the lower limit, the functionality of the base excision repair inhibitor tends to decrease, while if the chain length of the linker 2 is more than the upper limit, the expression efficiency in the case of expressing a fusion protein tends to decrease.

The linker 2 according to the present invention includes, for example, 10 amino acids described in pCMV_AncBE4max_P2A_GFP (ID: 112100) of Addgene, but is not limited to this.

### [Others]

The fusion protein according to the present invention may further contain a FLAG-tag for purification or detection, a nuclear localization signal (NLS) for transporting the TALE-nucleic acid base converting enzyme into cell nucleus, and the like at the N-terminus and/or C-terminus, and these may be contained in the N-terminal domain of the TALE as described above, or may be added to the N-terminus and/or C-terminus of the fusion protein.

The fusion protein according to the present invention can be obtained, for example, by transcribing expressing genes encoding the above-described TALE and nucleic acid base converting enzyme, as well as the linker 1, the base excision repair inhibitor, the linker 2, and other amino acids as necessary in an integrated manner. In addition, the fusion protein according to the present invention can be produced by employing and modifying a conventionally known method as appropriate, and can be obtained, for example, by a method that chemically synthesizes the fusion protein by using a commercially-available synthesizer based on the amino acid sequence, or a method that expresses the fusion protein by introducing a polynucleotide encoding the fusion protein or a vector expressing the fusion protein into the cell, as described in the method for producing a cell in which a target DNA is edited described below.

### (CRISPR-Cas9 system)

The CRISPR-Cas9 system according to the present invention contains a Cas9 protein which has lost part or all of a nuclease activity and a guide RNA thereof.

### [Cas9 protein]

In the CRISPR-Cas9 system according to the present invention, the Cas9 protein needs to be a Cas9 protein which has lost part of the nuclease activity (referred to as "nCas9" in the Specification) or a Cas9 protein which has lost all of the nuclease activity (referred to as "dCas9" in the Specification). In other words, Cas9 proteins typically contain a domain (RuvC domain) involved in cleavage of target strands and a domain (HNH domain) involved in cleavage of non-target strands. The Cas9 protein according to the present invention needs to have lost the nuclease activity of at least one of the domains due to introduction of mutation or the like.

In the case of SpCas9 protein (Cas9 protein derived from S. pyogenes), such mutation includes, for example, mutation into alanine of the 10th amino acid (aspartic acid) from the N terminus (D10A: mutation in the RuvC domain), mutation into alanine of the 840th amino acid (histidine) from the N terminus (H840A: mutation in the HNH domain), mutation into alanine of the 863rd amino acid (asparagine) from the N terminus (N863A: mutation in the HNH domain), mutation into alanine of the 762nd amino acid (glutamic acid) from the N terminus (E762A: mutation in the RuvCII domain), and mutation into alanine of the 986th amino acid (aspartic acid) from the N terminus (D986A: mutation in the RuvCIII domain). Besides, Cas9 proteins derived from various sources are known (for example, WO2014/131833), and any of these nCas9 and dCas9 can be used, but the Cas9 protein is not limited to these.

Note that the amino acid sequences and base sequences of Cas9 proteins are registered in a public database, for example, Genbank (http://www.ncbi.nlm.nih.gov) (for example, accession number: KX151730.1 and the like), and these can be used in the present invention. In addition, in the Cas9 protein, further mutation, for example, mutation for modifying the recognition of PAM sequences maybe introduced (Benjamin, P. et al., Nature 523, 481-485 (2015); Hirano, S. et al., Molecular Cell 61, 886-894 (2016)), and further a nuclear localization signal (NLS) for transporting into cell nuclei and the like may be added. In addition, such Cas9 protein may be any of those which are encoded in sequences contained in commercially-available plasmids.

Regarding the Cas9 protein in the CRISPR-Cas9 system, a guide RNA described below is designed such that the guide RNA-target sequence contains the complementary base of the target site of the target DNA. Note that in the present invention, the complementary base of the target site of the target DNA refers to a complementary base of one base of the target site on a complementary strand of the strand where the target site is present. In this way, the target site is specifically exposed by the helicase activity, which exposes a single-stranded DNA, in the target DNA, which is a double-strand, to improve the editing efficiency by using the nucleic acid base converting enzyme.

### [Guide RNA]

In the CRISPR-Cas9 system according to the present invention, the guide RNA is a combination of crRNA (CRISPR RNA) containing a base sequence (hereinafter, sometimes referred to as a "targeting base sequence") complementary to a sequence (guide RNA-target sequence) on the target DNA and tracrRNA (trans-activating crRNA). The crRNA further contains a base sequence capable of interacting (hybridizing) with the tracrRNA on the 3' side. On the other hand, the tracrRNA contains a base sequence capable of interacting (hybridizing) with the base sequence of part of the crRNA on the 5' side, the guide RNA forms double-stranded RNA which interacts with the Cas9 protein through the interaction of these base sequences. Hence, the guide RNA recognizes and binds to the guide RNA-target sequence of the target DNA, and guides the Cas9 protein, which forms a complex with the guide RNA, to the target DNA, and the Cas9 protein thus guided exposes the single-stranded DNA in the target site of the target DNA through its helicase activity.

The guide RNA of the CRISPR-Cas9 system according to the present invention may be a single guide RNA (sgRNA) containing crRNA and tracrRNA or may be a dual guide RNA composed of a crRNA fragment and a tracrRNA fragment.

In the target DNA, the editing of a base of the target site takes place at a position that is determined by the complementation of base-pair formation between the targeting base sequence of the guide RNA and the guide RNA-target sequence and a PAM sequence present on the 3' side of a complementary strand of the guide RNA-target sequence. The guide RNA of the CRISPR-Cas9 system according to the present invention is designed such that the guide RNA-target sequence becomes a sequence containing a complementary base of the target site, and the Cas9 protein can recognize the PAM sequence on the 3' side of the target site. In this way, the target site is specifically exposed by the helicase activity, which exposes a single-stranded DNA, in the target DNA, which is a double-strand, to improve the editing efficiency by using the nucleic acid base converting enzyme.

As the method for designing a PAM sequence and a guide RNA-target sequence of a Cas9 protein, various methods have been known, and the design may be determined by using, for example, E-CRISP (http://www.e-crisp.org/E-CRISP/), Zifit Targeter (http://zifit.partners.org/ZiFiT/) (Zing Finger consortium), CRISPR direct (http://crispr.dbcls.jp/) (University of Tokyo), CRISPR-P (http://cbi.hzau.edu.cn/crispr/) (Huazhong Agricultural University), Guide RNA Target Design Tool. (https://wwws.blueheronbio.com/external/tools/g RNA Src.jsp) (Blue Heron Biotech), and the like.

The CRISPR-Cas9 system according to the present invention can be obtained, for example, by simultaneously transcribing • expressing genes encoding the above-described Cas9 protein and guide RNA, respectively, as described in the method for producing a cell in which a target DNA is edited described below. The Cas9 protein and the guide RNA according to the present invention can be produced by employing and modifying conventionally known methods as appropriate, and can be obtained, for example, by a method that introduces polynucleotides encoding these or an expression vector containing polynucleotides into the cell.

### (Target DNA)

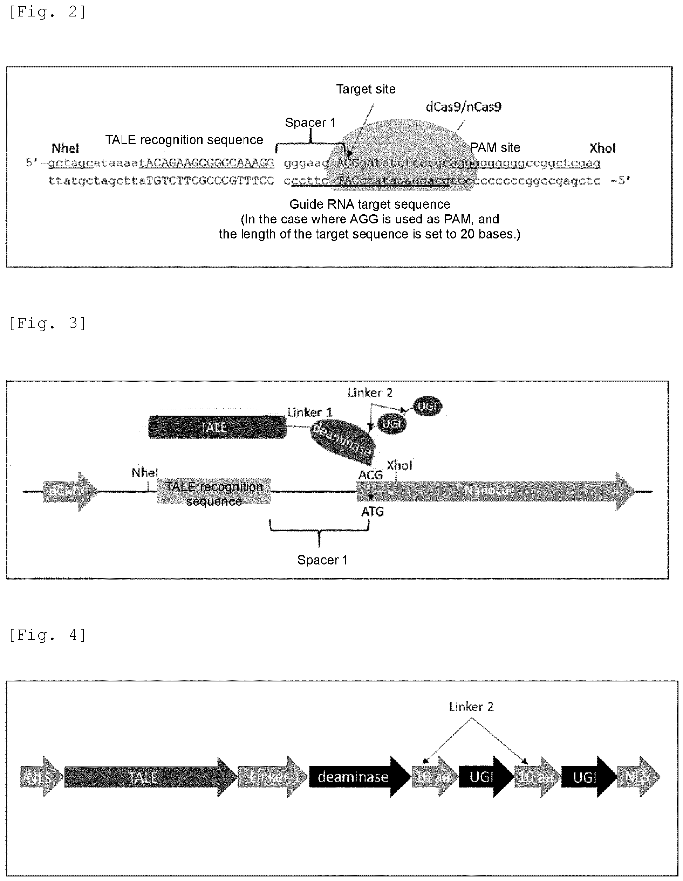

In the present invention, a DNA containing a target site to be subjected to objective DNA editing is referred to as a "target DNA". The target DNA according to the present invention is a double-stranded DNA, and in order to indicate correspondence with the above-described fusion protein and CRISPR-Cas9 system, it is assumed that at least one strand has a structure containing the TALE recognition sequence or a complementary sequence thereof, the spacer 1, the target site, and the PAM sequence in this order from the 5' side for the sake of convenience. Containing a complementary sequence of the TALE recognition sequence on the 5' side means that a complementary strand of the above strand contains the TALE recognition sequence. That is, the TALE recognition sequence may be set on the same strand as the target site or may be set on a complementary strand thereof. In addition, a complementary strand of the above strand is assumed to contain the guide RNA-target sequence so as to contain a complementary base of the target site. An example of the configuration of the target DNA according to the present invention is shown in Fig. 2, but the configuration is not limited to this.

The target site according to the present invention refers to one base to be edited by the nucleic acid base converting enzyme (preferably, deaminated by a deaminase) . However, this does not exclude cases where bases in the vicinity of either side of the one base (particularly, in a case where there is a base to be deaminated by a deaminase. preferably 1 to 10 bases, more preferably 1 to 5 bases, and further preferably 1 to 2 bases on each of the 5' side and 3' side) are further edited (deaminated in the case of a deaminase).

The TALE recognition sequence is a sequence recognized by the above TALE. The number of bases of the TALE recognition sequence is 10 to 30 bases, preferably 13 to 25 bases, and more preferably 15 to 20 bases. The TALE recognition sequence is selected to be present on the 5' side of the target site via a spacer 1 having a chain length of 7 to 31 bp. When the chain length of the spacer 1 is within the above range, it is possible to specifically and highly efficiently deaminate one base of the target site. The chain length of the spacer 1 is a length from a base adjacent to the 5' side of the base of the target site as the first base to a base adjacent to the 3' side of the TALE recognition sequence. The chain length of the spacer 1 is more preferably 10 to 31 bp, further preferably 10 to 28 bp, and even more preferably 13 to 25 bp.

The PAM sequence is a sequence recognized by the above Cas9 protein. The PAM sequence varies depending on the type of the Cas9 protein used. As typical PAM sequences, for example, a PAM sequence corresponding to the Cas9 protein (type II) derived from S. pyogenes is 5'-NGG, a PAM sequence corresponding to the Cas9 protein (type I-A1) derived from S. solfataricus is 5'-CCN, a PAM sequence corresponding to the Cas9 protein (type I-A2) derived from S. solfataricus is 5'-TCN, a PAM sequence corresponding to the Cas9 protein (type I-B) derived from H. walsbyl is 5'-TTC, a PAM sequence corresponding to the Cas9 protein (type I-E) derived from E. coli is 5'-AWG, a PAM sequence corresponding to the Cas9 protein (type I-F) derived from E. coli is 5'-CC, a PAM sequence corresponding to the Cas9 protein (type I-F) derived from P. aeruginosa is 5'-CC, a PAM sequence corresponding to the Cas9 protein (type II-A) derived from S. Thermophilus is 5'-NNAGAA, a PAM sequence corresponding to the Cas9 protein (type II-A) derived from S. agalactiae is 5'-NGG, a PAM sequence corresponding to the Cas9 protein derived from S. aureus is 5'-NGRRT or 5'-NGRRN, a PAM sequence corresponding to the Cas9 protein derived from N. meningitidis is 5'-NNNNGATT, and a PAM sequence corresponding to the Cas9 protein derived from T. denticola is 5'-NAAAAC. Note that as described above, it is also possible to modify the PAM recognition by modifying the Cas9 protein (for example, through introduction of mutation). This makes it possible to expand the options for the target site.

In the present invention, the PAM sequence is present on the 3' side of the target site, and the guide RNA-target sequence recognized by the guide RNA is determined depending on the position of the PAM sequence. In the present invention, the position of the complementary base of the target site in the guide RNA-target sequence is not particularly limited, but for example, is preferably between the 1st position and the 50th position, more preferably between the 1st position and the 30th position, and further preferably between the 1st position and the 25th position. Note that the position of the base in the guide RNA-target sequence is a position up to the complementary base of the base adjacent to the 5' side of the PAM sequence, where the position of the base at the 3' terminus of the guide RNA-target sequence is deemed as the 1st position. The upper limit and the lower limit for the position of the base depend on the length of the guide RNA-target sequence and can be adjusted by adjusting the length of the guide RNA.

The guide RNA-target sequence recognized by the guide RNA is selected to be a sequence containing the complementary base of the target site. By selecting the position of the guide RNA-target sequence in this way, it is possible to specifically and highly efficiently edit one base of the target site. The number of bases of the guide RNA-target sequence according to the present invention is preferably 12 to 50 base, more preferably 17 to 30 base, and further preferably 17 to 25 base.

The base sequence of such target DNA is not particularly limited, and the target for the DNA editing method of the present invention can be obtained by designing the targeting base sequences of the TALE and the guide RNA and modifying the recognition specificity of the PAM as necessary, so that the TALE recognition sequence or a complementary sequence thereof, the spacer 1, the complementary sequence of the guide RNA-target sequence, the target site contained in the complementary sequence, and the PAM sequence satisfy the above-described conditions.

Moreover, the target DNA according to the present invention may be a DNA present inside a cell (internal DNA) or a DNA present outside a cell depending on the object. The DNA present inside a cell may be an endogenous DNA or may be an exogenous DNA. The endogenous DNA includes genomic DNAs, and the exogenous DNA includes, for example, DNAs introduced in cells. The DNA present outside a cell may be a DNA derived from a cell or may be a DNA amplified or synthesized outside a cell.

### (Target DNA editing method)

In the target DNA editing method of the present invention, the fusion protein and the CRISPR-Cas9 system are brought into contact with the target DNA to edit the base in the target site of the target DNA by using the nucleic acid base converting enzyme activity of the fusion protein.

Once the fusion protein and the CRISPR-Cas9 system are brought into contact with the target DNA, the TALE of the fusion protein recognizes and binds to the TALE recognition sequence on the target DNA, and guides the nucleic acid base converting enzyme linked to the TALE to the target DNA. On the other hand, the guide RNA of the CRISPR-Cas9 system recognizes and binds to the guide RNA-target sequence on the target DNA, and guides the Cas9 protein, which forms a complex with the guide RNA, to the target DNA. In this way, since the Cas9 protein exposes the single-stranded DNA through the helicase activity in the target site, it is possible to efficiently cause substitution of the objective base (for example, deamination of the base) by using the nucleic acid base converting enzyme. In this way, substitution of one base occurs in the target site (for example, C_{→}U), and also for example, in the cell, the base of the strand opposite to the strand where the substitution has occurred is repaired to form a pair with the substituted base due to mismatch of the double-stranded DNA (for example, G_{→}A), or the base is substituted with another nucleotide during the repair (for example, U_{→}A, G), or deletion or insertion of one base or several tens of bases occurs, so that various mutations can be introduced.

Hence, the editing of DNA according to the present invention includes deletion of one or more nucleotides, substitution with other one or more nucleotides, or insertion of one or more nucleotides, or combinations of these mutations, in the target site converted by the nucleic acid base converting enzyme and the vicinity including the target site.

The target DNA editing method of the present invention may be conducted inside of a cell or may be conducted in a cell-free system. The "inside of a cell" where the target DNA editing method of the present invention is conducted may be inside of eukaryotic cell or may be inside of a prokaryotic cell, and is preferably inside of an eukaryotic cell. The eukaryotic cell includes, for example, animal cells (cells of mammals, fishes, birds, reptiles, amphibians, insects, and the like), plant cells, algal cells, and yeast. The prokaryotic cell includes, for example, E. coli, Salmonella, Bacillus subtilis, lactobacillus, and extreme thermophiles.

The "animal cells" include, for example, cells forming individuals of animals, cells forming organs • tissues extirpated from animals, cultured cells derived from tissues of animals, and the like. Specifically, the "animal cells" include, for example, germ cells such as oocytes and sperm; embryonic cells of embryos at various stages (for example, 1-cell embryos, 2-cell embryos, 4-cell embryos, 8-cell embryos, 16-cell embryo, morula embryos, and the like); stem cells such as induced pluripotent stem (iPS) cells and embryonic stem (ES) cells; somatic cells such as fibroblasts, hematopoietic cells, neurons, muscle cells, bone cells, liver cells, pancreatic cells, brain cells, and kidney cells, and the like. As oocytes for use in preparation of genome-edited animals, oocyte before fertilization and after fertilization can be used, but oocytes after fertilization, that is, fertilized eggs are preferable. Particularly preferably, fertilized eggs are from pronuclear stage embryos. As oocytes, cryopreserved oocytes can be thawed and used.

The "plant cells" include, for example, cells forming individuals of plants, cell forming organs and tissues separated from plants, cultured cells derived from tissues of plants, and the like. Organs and tissues of plants include, for example, leaves, stems, shoot apexes (growing points), roots, tubers, calluses, and the like.

In addition, the "cell-free system" in which the target DNA editing method of the present invention is conducted refers to a system in which there is no living cells (the eukaryotic cells, prokaryotic cells). The cell-free system according to the present invention is not particularly limited as long as it is a system in which the fusion protein and the CRISPR-Cas9 system can be brought into contact with the target DNA, but includes, for example, insides of buffer solutions ; insides of cell lysates and insides of cell extracts of the eukaryotic cell or prokaryotic cell, and the like.

The method for bringing the fusion protein and the CRISPR-Cas9 system into contact with the target DNA is not particularly limited. In the case of the inside of a cell, there is, for example, a method including: introducing a vector or the like that introduces the fusion protein and the CRISPR-Cas9 system into the cell or encodes these into a cell containing the target DNA to express these, as a method for producing a cell in which a target DNA is edited described below. In the case of the cell-free system, for example, a solution of the target DNA and a solution of the fusion protein and the CRISPR-Cas9 system may be mixed. A solvent for these solutions is not particularly limited, but for example, a buffer solution such as a phosphate buffer solution, a Tris buffer solution, a Good's buffer solution, or a boric acid buffer solution is preferable.

### <Method For producing cell in which target DNA is edited>

The method for producing a cell in which a target DNA is edited of the present invention is a method for producing a cell in which a target DNA is edited, comprising:
a step of introducing or expressing
   (1) a fusion protein containing a TALE and a nucleic acid base converting enzyme and
   (2) a CRISPR-Cas9 system containing a Cas9 protein which has lost part or all of a nuclease activity and a guide RNA thereof
into a cell or in a cell to bring the fusion protein and the CRISPR-Cas9 system into contact with a target DNA to edit a base in a target site of the target DNA by using a nucleic acid base converting enzyme activity of the fusion protein, wherein
a TALE recognition sequence recognized by the TALE in the fusion protein or a complementary sequence thereof is present on a 5' side of the target site via a spacer 1 having a chain length of 7 to 31 bp, and
a guide RNA-target sequence recognized by the guide RNA in the CRISPR-Cas9 system is present to contain a complementary base of the target site.

In the method for producing a cell in which a target DNA is edited (hereinafter, sometimes referred to simply as the "production method") of the present invention, the fusion protein, the CRISPR-Cas9 system, and the target DNA are as described in the above-described target DNA editing method of the present invention. The target DNA in the production method of the present invention is a genomic DNA, and the fusion protein and the CRISPR-Cas9 system can be designed depending on the objective of the editing of the genomic DNA.

In addition, in the production method of the present invention, the fusion protein and the CRISPR-Cas9 system are brought into contact with the target DNA in a cell by introducing the fusion protein and the CRISPR-Cas9 system into the cell in the form of protein, introducing the fusion protein and the CRISPR-Cas9 system into the cell in the form of polynucleotide, and/or introducing the fusion protein and the CRISPR-Cas9 system into the cell in the form of polynucleotide or introducing the fusion protein and the CRISPR-Cas9 system using expression vectors to express the fusion protein and the CRISPR-Cas9 system in the cell. Hence, as the fusion protein and the CRISPR-Cas9 system, the fusion protein and the Cas9 protein may be each independently introduced into a cell in the form of protein, or introduced into the cell in the form of RNA or DNA (polynucleotide) encoding the protein and expressed in the cell, or introduced into the cell in the form of vector (expression vector) expressing the protein and expressed in the cell. In addition, separately from this, the guide RNA may be introduced into the cell in the form of RNA, or introduced into the cell in the form of DNA (polynucleotide) encoding the RNA and expressed in the cell, or introduced into the cell in the form of vector (expression vector) expressing the RNA and expressed in the cell.

In the case where the fusion protein and the CRISPR-Cas9 system are introduced into a cell in the form of expression vector and expressed in the cell, for example, a vector expressing the fusion protein, a vector expressing the Cas9 protein, and a vector expressing the guide RNA each may be introduced into the cell, or a vector expressing two or more of these in combination may be introduced into the cell.

In addition, in the case where the fusion protein and the CRISPR-Cas9 system are introduced into a cell in the form of expression vector and expressed in the cell, polynucleotides encoding the fusion protein and the Cas9 protein may be each independently codon-optimized depending on the cell to introduce them. In addition, the expression vectors preferably contain a promoter and/or another control sequence operably linked to the polynucleotides to be expressed. Moreover, it is preferable that the expression vectors be capable of stably expressing proteins to be encoded without being incorporated in the host genome. Such expression vectors can be prepared in accordance with a conventionally known method as appropriate.

As the method for introducing proteins of the fusion protein and the CRISPR-Cas9 system, polynucleotides encoding the proteins, or vectors expressing the proteins into a cell, a known method for introducing proteins, DNA, or RNA fragments into a cell can be employed as appropriate depending on the type of the cell. Such method includes, for example, the electroporation method, the microinjection method, the particle gun method, the calcium phosphate method, the polyethyleneimine (PEI) method, the liposome method (the lipofection method), the DEAE-dextran method, cationic lipid-mediated transfection, viruses (adenovirus, lentivirus, adeno-associated virus, baculovirus, and the like), the agrobacterium method, the lithium acetate method, the spheroplast method, the heat shock method (the calcium chloride method, the rubidium chloride method), and the like. Such methods are described in many standard laboratory manuals such as "Leonard G. Davis et al., Basic methods in molecular biology, New York: Elsevier, 1986".

Once the fusion protein and the CRISPR-Cas9 system are introduced into a cell or expressed in the cell, the fusion protein and the CRISPR-Cas9 system come into contact with the target DNA in the cell, base substitution of the objective one base is caused in the target site by the target DNA editing mentioned in the above-described target DNA editing method of the present invention, and as a result a cell in which the target DNA is edited can be obtained.

The present invention also provides a method for preparing a non-human individual containing a cell in which the target DNA is edited. This method comprises a step of preparing a non-human individual from a cell obtained by the above-described production method. The non-human individual includes, for example, non-human animals and plants. The non-human animals include mammals (mice, rats, guinea pigs, hamsters, rabbits, monkeys, pigs, cattle, goats, sheep, and the like), fishes, birds, reptiles, amphibians, and insects. In the case of preparing a model animal, the mammal is preferably a rodent such as a mouse, a rat, a guinea pig, and a hamster, and is particularly preferably a mouse. The plants include, for example, grains, oil crops, forage crops, fruits, and vegetables. Specific examples of crops include, for example, rice, corn, banana, peanut, sunflower, tomato, turnip rape, tobacco, wheat, barley, potato, soybean, cotton plant, and carnation.

As the method for preparing a non-human individual from a cell in which the target DNA is edited, a known method can be used. In the case of preparing a non-human individual from a cell in an animal, a germ cell or a pluripotent stem cell is normally used. For example, an offspring can be obtained by microinjecting the fusion protein and the CRISPR-Cas9 system into an oocyte, and transplanting the oocyte thus obtained into uterus of a female non-human mammal brought into a pseudopregnancy state. In addition, in the case of plants, it has been known that the somatic cells have totipotency since a long time ago. For example, a plant in which desired DNA is edited can be obtained by microinjecting the fusion protein and the CRISPR-Cas9 system into a plant cell, and reproducing the plant from the plant cell thus obtained. In addition, it is also possible to obtain progeny or clones in which a desired DNA is edited from the non-human individual thus obtained.

The checking of the presence or absence of the target DNA editing and the determination of the genotype can be conducted based on conventionally known methods, and for example, the PCR method, the sequence determination method, the Southern blotting method, and the like can be used.

### <DNA editing system>

In addition, the present invention provides a DNA editing system comprising:
(1) a fusion protein containing a TALE and a nucleic acid base converting enzyme; and
(2) a CRISPR-Cas9 system containing a Cas9 protein which has lost part or all of a nuclease activity and a guide RNA thereof, for use in the above-described target DNA editing method of the present invention, the above-described method for producing a cell in which a target DNA is edited of the present invention, or the above-described method for preparing a non-human individual of the present invention.

The fusion protein and the CRISPR-Cas9 system are as mentioned in the above-described target DNA editing method and production method of the present invention. These may be each independently in the form of protein or RNA, or in the form of polynucleotide encoding the protein or RNA, or in the form of vector (expression vector) expressing the protein or RNA.

In the case of the form of vector, the present invention provides a DNA editing system comprising
(a) a vector containing an insertion site of a polynucleotide encoding TALE and a polynucleotide encoding a portion other than the TALE of the fusion protein;
(b) a polynucleotide encoding a Cas9 protein which has lost part or all of a nuclease activity, or a vector containing the polynucleotide; and
(c) a polynucleotide encoding a guide RNA of the Cas9 protein, or a vector containing the polynucleotide, or a vector containing an insertion site of the polynucleotide, in order for the user to be capable of designing the fusion protein and the CRISPR-Cas9 system depending on the target site of the target DNA. Note that each of the vectors of (a) to (c) contains an expression unit which enables each corresponding polynucleotide to be expressed.

The DNA editing system of the present invention may be a combination product composed of a combination of the fusion protein and the CRISPR-Cas9 system, or a kit comprising the combination. In the case of a kit, the kit may further comprise one or multiple additional reagents. Such additional reagents include, for example, a diluted buffer solution, a reconstitution solution, a wash buffer solution, a nucleic acid transfection reagent, a protein transfection reagent, a control reagent (for example, control deaminase), but are not limited to these. In addition, the kit may further comprise an instruction manual for implementing the methods of the present invention.

The elements included in the kit may be stored respectively in separate containers, or may be stored in the same container. The elements each may be stored in a container in an amount of a single use, or each may be stored in a single container in an amount of multiple uses. The elements each may be stored in a container in a dried form, or each may be stored in a container in the form of being dissolved in an appropriate solvent (a solvent containing abuffer solution, a stabilizer, a preservative, an antiseptic, and the like).

### [Examples]

Hereinafter, the present invention is described in further detail based on Examples; however, the present invention is not limited to the following Examples.

### 1. Preparation of reporter plasmids

Using pNLF1-C [CMV Hygro] (produced by PROMEGA Corporation, WI, USA) as a reporter, pNLF-M/A was obtained by converting the 71st and the 107th methionine codons (ATG) to alanine codons (ATA) by using the site-directed mutagenesis method. Subsequently, sequences (BEtag) shown in Table 1 given below were first inserted into the pNLF-M/A. The BEtag sequences each contained a TALE recognition sequence (the underline (*1) in Table 1: 5'-tACAGAAGCGGGCAAAGG-3'; lower-case letters indicate thymine recognized by the N-terminal domain of TALE), a target codon (the underline (*2) in Table 1: 5'-ACG-3'), and a spacer 1 sequence between these, and the length of the spacer 1 sequence was set to 7 bp, 13 bp, 19 bp, 25 bp, or 31 bp, containing the base (A) at 5' of the target codon. To each BEtag sequence (BEtag-7 to 31bp: SEQ ID NOs: 1 to 5), an oligonucleotide designed such that a NheI recognition site and a SbfI recognition site were able to be added at the two ends was annealed, and inserted into pNLF-M/A treated with NheI and SbfI to obtain pNLF-BEtag (pNLF-BEtag-7bp to 31bp).

Note that the TALE recognition sequence was a right TALE sequence of TALEN for the human adenomatous polyposis coli gene whose activity in cells has been confirmed (Sakuma et al., "Repeating pattern of non-RVD variations in DNA-binding modules enhances TALEN activity.", Sci. Rep., 3: 3379 (2013)).

Moreover, in order to insert the PAM sequence (5'-NGG-3') into pNLF-BEtag, an oligonucleotide having a sequence (PAM: SEQ ID NO: 6) containing a PAM site as shown in Table 1 given below and an oligonucleotide in which 5'-TCGA-3' was added to the 5' terminus of the antisense sequence thereof were annealed, and inserted into each pNLF-BEtag treated with EcoRV and XhoI to prepare reporter plasmids (pNLF-BEtag-7bp-PAM, pNLF-BEtag-13bp-PAM, pNLF-BEtag-19bp-PAM, pNLF-BEtag-25bp-PAM, pNLF-BEtag-31bp-PAM) .

Since the PAM site (the underline (*3) in Table 1) contains nine consecutive "G", the position of the PAM sequence can be shifted downstream by one base each time by designing the guide RNA such that the complementary sequence of the guide RNA-target sequence positioned on the 5' side of the PAM site is shifted to the 3' side by one base each time. In this way, PAM sequences at eight positions (PAM1 to 8 in Table 7 given below) can be set. Each sequence inserted into the pNLF-M/A plasmid is shown in Table 1 given below.

**[Table 1]**

| Products | Sequence (5'-3') | SEC ID NO |
|---|---|---|
| BEtag-7bp | CATAAAATACAGAAGCGGGCAAAGG*¹GGGAAGACG*²GATATCTCC | 1 |
| BEtag-13bp | CATAAAATACAGAAGCGGGCAAAGG*¹CTATTTGGGAAGACG*²GATATCTCC | 2 |
| BEtag-19bp | CATAAAATACAGAAGCGGGCAAAGG*²ATTAGTCTATTTGGGAAGACG*²GATATCTCC | 3 |
| BEtag-25bp | CATAAAATACAGAAGCGGGCAAAGG*¹TTCAGGATTAGTCTATTTGGGAAGACG*²GATATCTCC | 4 |
| BEtag-31bp | CATAAAATACAGAAGCGGGCAAAGG*¹AGTCTATTCAGGATTAGTCTATTTGGGAAGACG*²GATATCTCC | 5 |
| PAM | ATCTCCTGCAGGGGGGGGG*³CCGGC | 6 |

| | | |
|---|---|---|
| *1: TALE recognition sequence *2: target codon containing target base *3: PAM site | | |

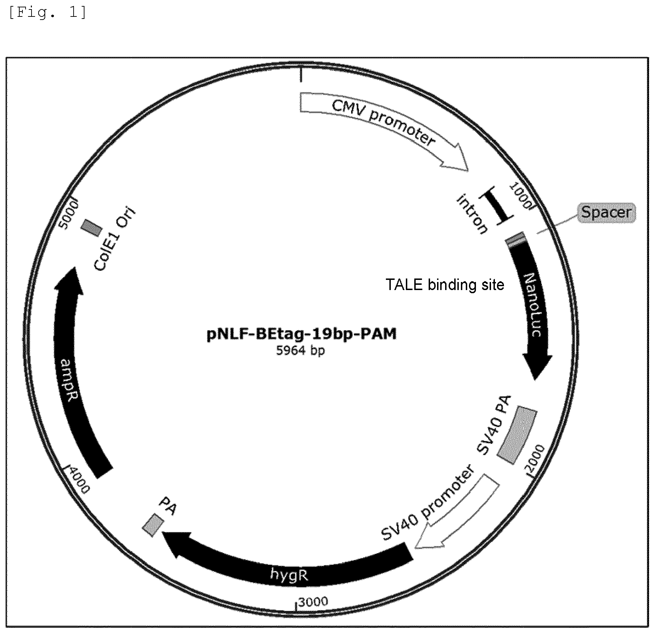

A concept diagram showing the structure of the reporter plasmid thus completed is shown in Fig. 1. In addition, Fig. 2 shows the sequence from the NheI recognition site to the XhoI recognition site, which contains the TALE recognition sequence, the spacer 1, the target site, the PAM site, and the guide RNA-target sequence. In this reporter plasmid, the target codon is located at a position corresponding to the start codon of NanoLuc luciferase, and according to such reporter plasmid, once ACG, which is the target codon, is converted to ATG (C, which is the target base (the target site), is substituted with T) by the TALE-deaminase bound to the TALE recognition sequence, NanoLuc luciferase is expressed (Fig. 3).

### 2. Preparation of a TALE vector set and TALE expression Plasmids

The sequence and configuration of the TALE vector set was constructed in accordance with Sakuma et al. (2013) such that the TALE repeat unit corresponds to the TALE recognition sequence of the reporter plasmid prepared in the above 1. First, sequences (module sequences) (16 types in total: 1HD to 4HD, 1NG to 4NG, 1NI to 4NI, and 1NN to 4NN) were prepared through artificial DNA synthesis by adding modifications (particularly, the 4th and the 32nd) to module sequences (non-repeat-variable di-residue (non-RVD)) other than the sequences encoding four types of variable residues (RVD: HD, NG, NI, NN) formed by the 12th and 13th, two amino acids, and further adding restriction enzyme BsAI recognition sites to the two terminuses thereof. Subsequently, these module sequences were inserted into pEX-A2J2 (produced by Eurofins Genomics K.K., Tokyo, Japan) to prepare module plasmid sets (16 types in total: pEX1HD to pEX4HD, pEX1NG to pEX4NG, pEX1NI to pEX4NI, and pEX1NN to pEX4NN) .

In addition, DNA sequences FUS2_aXX (7 types in total: XX=1a, 2a, 2b, 3a, 3b, 4a, 4b) and FUS2_b(1-4) (4 types in total) forming array plasmids were prepared through artificial DNA synthesis. These were inserted into pCR8/GW/TOPO (produced by Thermo Fisher Scientific Inc., Waltham, MA, USA) to prepare pCR8_FUS2_aXX and pCR8_FUS_b(1-4). Subsequently, these were used as trap vectors in the initial assemble step (step 1) in the Platinum Gate system described in Sakuma et al. (2013), and array plasmids in which TALE sequences were linked were prepared in accordance with the method described in the same document.

Subsequently, destination vectors (TALE-WT, TALE-63, TALE-47) was prepared by inserting one of the module sequences and a sequence encoding the C-terminal domain of the TALE following the 3' terminus of this into pcDNA 3.1s obtained by removing a drug-resistant gene expression unit from pcDNA 3.1(+) (produced by Thermo Fisher Scientific Inc.) in addition to a sequence encoding the N-terminal domain of the TALE, which was prepared through artificial DNA synthesis. The C-terminal domains of the TALE were synthesized through artificial DNA synthesis by using three types of wild type (WT: the number of amino acids of the C-terminal domain=180), a C-terminal domain having a number of amino acids of 63 (63), and a C-terminal domain having a number of amino acids of 47 (47) and referring to the sequence of the C-terminal domain contained in pTALETF_v2 (ID: 32185 to 32188) of Addgene as the sequence encoding "WT", the sequence of the C-terminal domain contained in pTALEN_v2 (ID: 32189 to 32192) of Addgene as the sequence encoding "63", and the sequence of the C-terminal domain contained in ptCMV-153/47-VR-NG (Addgene ID: 50704) of Addgene as the sequence encoding "47". In addition, the N-terminal domains of the TALE were synthesized through artificial DNA synthesis by referring to WT: the sequence of the N-terminal domain contained in pTALETF_v2 (ID: 32185 to 32188) of Addgene; 63: the sequence of the N-terminal domain contained in ptCMV-136/63-VR-HD (ID: 50699) of Addgene; and 47: the sequence of the N-terminal domain contained in ptCMV-153/47-VR-HD (ID: 50703) of Addgene, respectively, in correspondence to these C-terminal domains. TALE expression plasmids were prepared by the Golden Gate method using the array plasmids and destination vectors prepared as described above, in accordance with the method described in Sakuma et al. (2013) .

### 3. Preparation of TALE-deaminase expression plasmids (1) (1) 104 Amino acid linker series

First, a AncBE4max expression plasmid and a Target-AID expression plasmid were prepared by respectively inserting a AncBE4max gene (Koblan et al., "Improving cytidine and adenine base editors by expression optimization and ancestral reconstruction.", Nat Biotechnol, 36, p. 843-846 (2018)) and a Target-AID gene (Nishida et al., "Targeted nucleotide editing using hybrid prokaryotic and vertebrate adaptive immune systems.", Science 353, aaf8729 (2016)), which were prepared through artificial DNA synthesis, between the BamHI recognition site and the EcoRV recognition site of the pcDNA 3.1s.

Subsequently, TALE-deaminase expression plasmids having 104 amino acid linker 1 were prepared by the following method. Specifically, double repeat sequences of the Anc689 deaminase gene (Anc689 deaminase: primer SEQ ID NO: 7 to 8), the 10 amino acid linker 2, and the UGI gene (10aa linker-β-lactamase: primer SEQ ID NOs: 11 to 12) were respectively amplified by PCR using primers described in Table 2 given below with AncBE4max gene of the above-described AncBE4max expression plasmid as a template. In addition, PmCDAl deaminase gene (PmCDAl: primer SEQ ID NOs: 9 to 10) and a sequence encoding 104 amino acid linker 1 (104aa linker: primer SEQ ID NOs: 13 to 17) were amplified by PCR using the primers described in Table 2 given below with Target-AID gene of the above-described Target-AID expression plasmid as a template. Moreover, a sequence from β-lactamase to a sequence encoding the N-terminal domain (WT, 63, or 47) of the TALE (primer SEQ ID NOs: 22 to 25) inclusive was amplified by PCR. The primers used for constructing TALE-deaminase expression plasmids and SEQ ID NOs thereof are shown in Table 2 given below. In Table 2, the codes following for of 104aa linker are such that, for example, "WT-104-BE4" indicates that this sequence is a sequence encoding linker 1 for TALE-deaminase in which the C-terminal domain of the TALE is WT and which contains the 104 amino acid linker 1 and the Anc689 deaminase (BE4) . Note that in each Table given below, "AID" does not indicate that the deaminase is AID but indicates that the deaminase is PmCDAl derived from "Target-AID".

**[Table 2]**

| Products | Forward Primer (5'-3') | SEQ ID NO | Reverse Primer (5'-3') | SEQ ID NO |
|---|---|---|---|---|
| Anc698 deaminase | anc689deaminase_F | 7 | anc689deaminase_R_inf10aa | 8 |
| PmCDAl | PmCDA1_F | 9 | PmCDA1_R_inf10aa | 10 |
| 10aa linker-β-lactamase | 10aa_F | 11 | Amp_F | 12 |
| 104aa linker for WT-104-BE4 | 104aa_F_infWT | 13 | AID_R3_infAnc689 | 15 |
| 104aa linker for 47-104-BE4 | 104aa_F_inf47 | 14 | | |
| 104aa linker for WT-104-AID | 104aa_F_infWT | 13 | AID_R3_infCDA1 | 17 |
| 104aa linker for 63-104-AID | 104aa_F_inf63 | 16 | | |
| 104aa linker for 47-104-AID | 104aa_F_inf47 | 14 | | |
| TALE_WT-β-lactamase | TALE_WT_R | 22 | Amp_R | 25 |
| TALE_63-β-lactamase | TALE_63_R | 23 | | |
| TALE_47-β-lactamase | TALE_47_R | 24 | | |

Each deaminase, a sequence encoding the 104 amino acid linker 1, a double repeat sequence of the 10 amino acid linker 2 and the UGI gene were inserted between a sequence from β-lactamase to a sequence encoding the N-terminal domain (WT, 63, or 47) of the TALE inclusive, which was prepared in the above 2., and a downstream (3' side) sequence encoding the C-terminal domain (WT, 63, or 47) of the TALE by the In-Fusion method (TaKaRa Bio Inc, Shiga, Japan) to prepare a plasmid (TALE-deaminase expression plasmid) expressing TALE-deaminase in which TALE (N-terminal domain (WT, 63, or 47)-TALE repeat domain-C-terminal domain (WT, 63, or 47)), 104 amino acid linker 1 (104aa), deaminase (Anc689 deaminase (BE4) or PmCDAl deaminase (AID)), 10 amino acid linker 2 (10aa), UGI gene (UGI), 10 amino acid linker 2 (10aa), and UGI gene (UGI) were bound in this order from the N-terminus. A concept diagram showing the structure of the TALE-deaminase is shown in Fig. 4.

### (2) 12 Amino acid linker series

In addition, TALE-deaminase expression plasmids having 12 amino acid linker 1 (12aa) in place of the above 104 amino acid linker 1 were prepared by the following method. Specifically, sequences encoding 12 amino acid linker 1 were prepared by annealing oligonucleotides described in Table 3. In Table 3, the codes following for of 12aa linker( SEQ ID NOs: 18 to 21) are such that, for example, "TALE_WT-12-AID" indicates that this sequence is a sequence encoding linker 1 for TALE-deaminase in which the C-terminal domain of the TALE is WT and which contains the 12 amino acid linker 1 and the PmCDAl deaminase (AID) . The 12 amino acid linker 1 is composed of 12 amino acids described in Yang L et al., "Engineering and optimising deaminase fusions for genome editing.", Nat Commun 7 13330 (2016). Subsequently, a portion other than the sequence encoding 104 amino acid linker 1 of the above-described TALE-deaminase expression plasmid was amplified by inverse PCR using each of primers (SEQ ID NOs: 9, 22 to 24) shown in Table 4 given below, and the sequence encoding 12 amino acid linker 1 was inserted by the In-Fusion method. Sequences encoding 12 amino acid linker 1 and SEQ ID NOs thereof are shown in Table 3 given below, and the primers used for inverse PCR and SEQ ID NOs thereof are shown in Table 4 given below.

**[Table 3]**

| Products | Sense oligonucleotide | SEQ ID NO | Antisense oligonucleotide | SEQ ID NO |
|---|---|---|---|---|
| 12 aa linker for TALE_WT-12-AID | 12aa_infWT | 18 | 12aa_infCDA1 | 21 |
| 12 aa linker for TALE_63-12-AID | 12aa_inf 63 | 19 | | |
| 12 aa linker for TALE_47-12-AID | 12aa_inf47 | 20 | | |

**[Table 4]**

| Final Products | Forward Primer ( 5'-3' ) | SEQ ID NO | Reverse Primer ( 5'-3' ) | SEQ ID NO |
|---|---|---|---|---|
| TALE_WT-12-AID | PmCDA1_F | 9 | TALE_WT_R | 22 |
| TALE_63-12-AID | | | TALE_63_R | 23 |
| TALE_12-12-AID | | | TALE_47_R | 24 |

### (3) Amino acids in length adjustment linker series

Furthermore, TALE-deaminase expression plasmids encoding, in place of 104 amino acid linker 1, amino acid linkers 1 which were obtained by deleting amino acids in the order from the C-terminal side of the 104 amino acid linker 1 so as to have 12, 24, 36, 48, 60, or 84 amino acids in length in TALE-47-104-AID were prepared by the following method. Specifically, a portion other than the sequence encoding the amino acids to be deleted of the above-described TALE-deaminase expression plasmid was amplified by inverse PCR using primers (SEQ ID NOs: 9, 26 to 31) described in Table 5 given below, and caused to self-ligate by the In-Fusion method. The primers used for preparing the amino acid linker 1 deletion mutants of TALE-47-104-AID and SEQ ID NOs thereof are shown in Table 5 given below.

**[Table 5]**

| Final Products | Forward Primer (5'-3') | SEQ ID NO | Reverse Primer ( 5'-3' ) | SEQ ID NO |
|---|---|---|---|---|
| TALE_47-12'-AID | PmCDA1_F | 9 | 12aa_R_infCDA1 | 26 |
| TALE_47-24'-AID | | | 24aa_R_infCDA1 | 27 |
| TALE_47-36'-AID | | | 36aa_R_infCDA1 | 28 |
| TALE_47-48'-AID | | | 48aa_R_infCDA1 | 29 |
| TALE_47-60'-AID | | | 60aa_R_infCDA1 | 30 |
| TALE_47-84'-AID | | | 84aa_R_infCDA1 | 31 |

In addition, as negative controls (NC) of TALE-deaminases, plasmids (TALE-deaminase-NC expression plasmids) expressing TALE-deaminase-NC corresponding to the respective TALE-deaminases were prepared in the same manner as described above except that TALE-deaminase-NC constructed to recognize and bind to the NC sequence :5'-tTGCGCGTATAGTCGCG-3' (SEQ ID NO: 32) in place of the TALE recognition sequence of the reporter plasmids prepared in the above-described 1. was expressed so that the TALE repeat units did not bind to the human genome.

A list of the configurations of the TALE-deaminases and negative controls thereof (TALE-deaminase-NC) of the prepared expression plasmids as well as SEQ ID NOs showing the sequences (SEQ ID NOs of base sequences, SEQ ID NOs of amino acid sequences) are shown in Table 6.

**[Table 6]**

| TALE-deaminase | | | | | | TALE-deaminase-NC (negative control) | | | | | |
|---|---|---|---|---|---|---|---|---|---|---|---|
| ID | TALE recognitio n sequence | TALE C-termina l domain | Length of linker 1 (a.a.) | Deaminase | SEQ ID NO | ID | TALE recognitio n sequence | TALE C-termina l domain | Length of linker 1 (a.a. ) | Deaminas e | SEQ ID NO |
| WT-104-BE 4 | Human | WT | 104 | Anc689 | 33, 34 | WT-104-BE 4-NC | Non-human | WT | 104 | Anc689 | 35, 36 |
| 47-104-BE 4 | | 47 | | | 37, 38 | 47-104-BE 4-NC | | 47 | | | 39, 40 |
| WT-104-AI D | | WT | 104 | PmCDAl | 41, 42 | WT-104-AI D-NC | | WT | 104 | PmCDAl | 43, 44 |
| 63-104-AI D | | 63 | | | 45, 46 | 63-104-AI D-NC | | 63 | | | 47, 48 |
| 47-104-AI D | | 47 | | | 49, 50 | 47-104-AI D-NC | | 47 | | | 51, 52 |
| WT-12-AID | | WT | 12 | PmCDAl | 53, 54 | WT-12-AID -NC | | WT | 12 | PmCDAl | 55, 56 |
| 63-12-AID | | 63 | | | 57, 58 | 63-12-AID -NC | | 63 | | | 59, 60 |
| 47-12-AID | | 47 | | | 61, 62 | 47-12-AID -NC | | 47 | | | 63, 64 |
| 47-12' to 84'-AID | | 47 | 12, 24, 36, 48, 60 or 84 | PmCDAl | - | 47-12' to 84'-AID-N C | | 47 | 12, 24, 36, 48, 60 or 84 | PmCDAl | - |
| AncBE4max | - | - | - | Anc689 | - | - | | | | | |

### 4. Preparation of Cas9 expression plasmids

First, a Cas9 expression plasmid pX330_BS was prepared by synthesizing a region from the U6 promoter to the BGH poly A addition sequence of the pX330 (Addgene, Cambridge, MA; Plasmid 42230) through artificial DNA synthesis, and inserting the region between the EcoRV recognition site and the BamHI recognition site of the pBlueScript II (SK+) (Stratagene, La Jolla, CA, USA) by the In-Fusion method. Subsequently, a nickase-type nCas9 (D10A) expression plasmid and a dCas9 (D10A+H840A) expression plasmid having no cleavage activity were prepared by conducting the site-directed mutagenesis method by PCR on the Cas9 gene of the pX330_BS.

### 5. Preparation of guide RNA expression plasmids (1)

First, a plasmid (pX330_BS-Δ Cas9) was prepared by treating the pX330_BS prepared in the above 4. with XbaI and NotI to remove the Cas9 gene, and conducting self-ligation after fill-in reaction. Subsequently, oligonucleotides designed to correspond to the target sequences of the guide RNAs shown in Table 7 given below (Table 7 shows complementary sequences of the target sequences (on the antisense strand) of the guide RNAs) (sequences other than those underlined in Table 7) were annealed, and inserted into the pX330_BS-Δ Cas9 by BpiI treatment and ligation to prepare plasmids (guide RNA expression plasmids 1 to 8) expressing the respective guide RNAs (sgRNA-1 to 8) . The complementary sequences (other than those underlined) of the target sequences of the guide RNAs and the PAM sequences (underlined) of Cas9 are shown in Table 7 given below.

**[Table 7]**

| Position of PAM sequence | Guide RNA | complementary sequence of target sequence of guide RNA and PAM sequence | SEQ ID NO |
|---|---|---|---|
| PAM1 | sgRNA-1 | GGAAGACGGATATCTCCTGC AGG | 65 |
| PAM2 | sgRNA-2 | GAAGACGGATATCTCCTGCA GGG | 66 |
| PAM 3 | sgRNA-3 | AAGACGGATATCTCCTGCAG GGG | 67 |
| PAM 4 | sgRNA-4 | AGACGGATATCTCCTGCAGG GGG | 68 |
| PAM 5 | sgRNA-5 | GACGGATATCTCCTGCAGGG GGG | 69 |
| PAM 6 | sgRNA-6 | ACGGATATCTCCTGCAGGGG GGG | 70 |
| PAM7 | sgRNA-7 | CGGATATCTCCTGCAGGGGG GGG | 71 |
| PAM8 | sgRNA-8 | GGATATCTCCTGCAGGGGGG GGG | 72 |

| | | | |
|---|---|---|---|
| Underline: PAM sequence | | | |

### 6. Transformation to HEK cells (1)

HEK293 T-cells grown in a DMEM medium containing 10% FBS were incubated in the respective wells of a 96-well plate with 5×10⁴ cells in each well. Into the HEK293 T-cells, 75 ng of one of the TALE-deaminase expression plasmids and the TALE-deaminase-NC expression plasmids described in Table 6; 50 ng of the nCas9 expression plasmid or the dCas9 expression plasmid prepared in the above 4.; 10 ng of one of the guide RNA expression plasmids 1 to 8 and the pX330_BS-Δ Cas9 (with no guide RNAs) prepared in the above 5.; 20 ng of one of the reporter plasmids prepared in the above 1.; and 5 ng of pGL4.54 (produced by PROMEGA Corporation), which was a reference plasmid, were introduced by using Lipofectamine LTX (produced by Thermo Fisher Scientific Inc.). The reference plasmid is a plasmid expressing firefly luciferase (Flue) .

In addition, as positive controls, 75 ng of the AncBE4max expression plasmid prepared in the above 3.; 10 ng of one of the guide RNA expression plasmids 1 to 8 and pX330_BS-Δ Cas9 (with no guide RNAs) prepared in the above 5.; 20 ng of one of the reporter plasmids prepared in the above 1.; and 5 ng of pGL4.54 were combined and introduced into the HEK293 T-cells.

### 7. Measurement of NanoLuc luciferase activity values

The medium was removed after culturing for 24 hours since transformation, followed by washing with PBS(-), and thereafter the cells were dissolved by treatment using Passive Lysis Buffer (produced by PROMEGA Corporation) to obtain cell lysates. The cell lysates were diluted to 100 times with DMEM media, and NanoLuc luciferase activity scores and firefly luciferase activity scores were measured with TriStar S LB942 plate reader (produced by Berthold Technologies, Bad Wildbad, Germany) by using Nano-Glo Dual-Luciferase Reporter Assay System (produced by PROMEGA Corporation).

For the negative control of each score, NanoLuc luciferase activity score of cells in which the TALE-deaminase-NC expression plasmid was introduced was measured. Note that for the positive controls (cells in which the AncBE4max expression plasmid was introduced), activity score of cells in which the pX330_BS-Δ Cas9 expression plasmid was introduced (cells expressing no guide RNA) was measured as the negative control (AncBE4max-NC).

The quantification of the activity was conducted as follows. First, each NanoLuc luciferase activity score was standardized by using the firefly luciferase activity score of the reference plasmid. Subsequently, by using the standardized activity score, the activity score of the cells in which the TALE-deaminase-NC expression plasmid was introduced was subtracted from the activity score of the cells in which the TALE-deaminase expression plasmid was introduced to obtain the activity value of the TALE-deaminase activity. In addition, AncBE4max-NC was subtracted from the activity score of the cells in which the expression plasmid of AncBE4max was introduced to obtain the AncBE4max activity value. Furthermore, in order to compare the TALE-deaminase activity between the tests, the TALE-deaminase activity value was represented as a relative activity as the AncBE4max activity value under PAM2 condition, which is described later, was deemed as 1. Each test was conducted repeatedly at least three times, and a standard deviation was added to the average value to obtain a graph.

### <Test 1> Effects of the position of the PAM sequence on the activity of base Editors

In order to examine the position of the PAM sequence where AncBE4max, which was an existing Base editor, exhibited the highest activity as the positive control, the position of the PAM sequence was shifted to the 3' side (downstream) by one base each time (PAM1 to PAM8), and the base editing activity was checked.

It is known that in AncBE4max, in the case where the position of the PAM sequence is set to the 21st to 23rd positions in a target DNA sequence, the position of a target base having a high base editing activity becomes the 4th to 8th positions in the same target DNA sequence (Rees, H. A. and Liu, D. R., Nat Rev Genet 19, 770-788 (2018)). In the reporter used in this study, the position of the PAM sequence can be changed from PAM1 to PAM8 described in Table 7 by designing the reporter such that the target sequence of the guide RNA changes as described above. Hence, the position of the target base is changed accordingly.

The AncBE4max expression plasmid prepared in the above 3., one of the guide RNA expression plasmids 1 to 8 prepared in the above 5., the reporter plasmid (spacer 1: 19 bp) prepared in the above 1., and pGL4.54, which was the reference plasmid, were combined and introduced into the HEK293 T-cells in the above 6., and AncBE4max activity values were obtained by the above 7. In addition, as the negative control, pX330_BS-Δ Cas9 was used in place of the guide RNA expression plasmid.

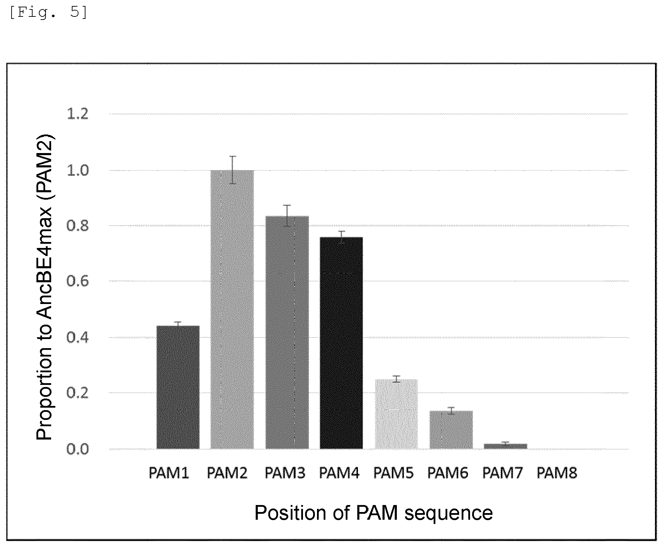

Results are shown in Fig. 5. In Fig. 5, on the premise that the AncBE4max activity value when the guide RNA expression plasmid 2 was used, that is, in PAM2 was deemed as 1.0, and the AncBE4max activity values when the guide RNA expression plasmids 1 to 8 were used, that is, in PAM1 to 8 are shown. As shown in Fig. 5, it was found that the AncBE4max activity value exhibited the maximum activity in PAM2. Hence, the AncBE4max activity value in PAM2 was determined as positive control for the following activity evaluation.

### <Test 2> Effects of the length of the spacer 1 on the activity of TALE-deaminases

### (BE4 series)

For two types of TALE-deaminases (WT-104-BE4, 47-104-BE4) in which the C-terminal domain of the TALE was WT or 47, the amino acid linker 1 had 104 amino acids, and the deaminase was BE4 (Anc689), the effects of the length of the spacer 1 of the reporter on the activity were checked with the position of the PAM sequence being fixed to PAM2. The length of the spacer 1 was set to 7 bp, 13 bp, 19 bp, 25 bp, and 31 bp. In addition, as Cas9/guide RNA, three conditions where nCas9 (D10A) and the guide RNA were expressed (nCas9), where dCas9 (D10A+H840A) and the guide RNA were expressed (dCas9), and where nCas9 was expressed and the guide RNA was not expressed (nCas9/no guide) were studied.

The TALE-deaminase expression plasmid (WT-104-BE4 or 47-104-BE4) prepared in the above 3. (1) or a TALE-deaminase-NC expression plasmid corresponding to this, the nCas9 expression plasmid or dCas9 expression plasmid prepared in the above 4., the guide RNA expression plasmid 2 prepared in the above 5. or pX330_BS-Δ Cas9, the reporter plasmid (spacer 1: 7 to 31 bp) prepared in the above 1., and pGL4.54, which was the reference plasmid, were combined and introduced into the HEK293 T-cells in the above 6., and TALE-deaminase activity values (proportions to AncBE4max activity values) were obtained by the above 7.

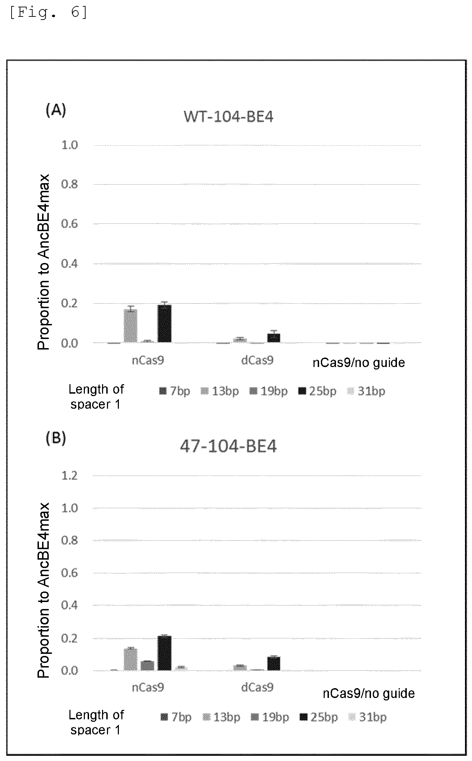

Results are shown in Fig. 6. As shown in Fig. 6, in any of the TALE-deaminases (BE4), in the case where there was no guide RNA, that is, in the case where nCas9 was not bound in the vicinity of the target base, no activity was observed. In addition, the activity was higher in the case where nCas9 was used and nicks were induced in the bottom strand than in the case where dCas9 was used and no nicks were induced. Regarding the length of the spacer 1, higher activity was exhibited when the length was 13 bp and 25 bp, and even higher activity was exhibited when the length was 25 bp. Regarding the C-terminal domain of the TALE, activity was higher in 47 (0.2910.03) than in WT (for example, in the case of nCas9 and the length of the spacer 1: 25 bp, 0.19±0.01).

### (AID series)

For six types of TALE-deaminases (WT-104/12-AID, 63-104/12-AID, 47-104/12-AID) in each of which the C-terminal domain of TALE was WT, 63, or 47, the amino acid linker 1 had 104 amino acids or 12 amino acids, and the deaminase was AID (PmCDA1), the effects of the length of the spacer 1 of the reporter on the activity were examined in the same manner as in the case of BE4 described above.

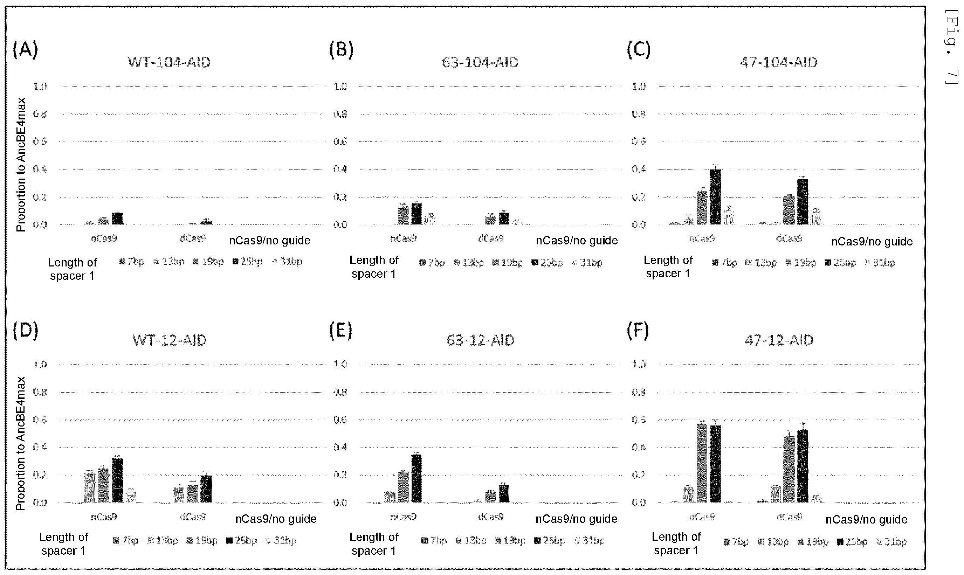

Results are shown in Fig. 7. As shown in Fig. 7, in any of the TALE-deaminases (AID), in the case where there was no guide RNA, that is, in the case where nCas9 was not bound in the vicinity of the target base, no activity was observed. In addition, in WT-104-AID, 63-104-AID, WT-12-AID, and 63-12-AID, the activity was higher in the case where nCas9 was used and nicks were induced in the bottom strand than in the case where dCas9 was used and no nicks were induced in the same manner as in the case where the deaminase was BE4. On the other hand, in 47-104-AID and 47-12-AID, the effect of nicks was not so significant.

Regarding the length of the spacer 1, higher activity was exhibited when the length was 25 bp in all of the TALE-deaminases (AID), and higher activity was similarly exhibited also when the length was 19 bp in 63-104-AID and 47-12-AID. Regarding the C-terminal domain of the TALE, in the case of the 104 amino acid linker 1, activity was such that WT (for example, in the case of nCas9 and the length of the spacer 1: 25 bp, 0.09±0.00), 63 (0.16±0.01), 47 (0.4±0.03) (FIGs. 7-A, B, C) ; and in the case of the 12 amino acid linker 1, activity was WT (0.3210.02), 63 (0.3810.03), 47 (0.57±0.02) (FIGs. 7-D, E, F). Hence, in the case where the length of the amino acid linker 1 is the same, 47>63>WT. In addition, in the case where comparison was made among those having the same C-terminal domain of the TALE, activity was higher with the 12 amino acid linker 1 than with the 104 amino acid linker 1. The TALE-deaminase (AID) exhibited the highest activity was 47-12-AID (for example, in the case of nCas9 and the length of the spacer 1: 19 bp, 0.57±0.2).

### <Test 3> Effects of the position of the PAM sequence on the activity of TALE-deaminases

The length of the spacer 1 of the reporter was fixed, and the position of the PAM sequence was shifted to the 3' side (downstream) by one base each time, and the effects of the position of the PAM sequence (that is, corresponding to the position of Cas9) on the activity of the TALE-deaminase were examined. As the length of the spacer 1, the study was conducted for 13 bp, 19 bp, 25 bp with which the activity was relatively high when the activity was evaluated in Test 2.

The TALE-deaminase expression plasmid (AID series: WT-104/12-AID, 63-104/12-AID, 47-104/12-AID) prepared in the above 3.(1) (2) or a TALE-deaminase-NC expression plasmid corresponding to this, the nCas9 expression plasmid prepared in the above 4., the guide RNA expression plasmids 1 to 8 prepared in the above 5., the reporter plasmid (spacer 1: 13, 19, or 25 bp) prepared in the above 1., and pGL4.54, which was the reference plasmid, were combined and introduced into the HEK293 T-cells in the above 6., and TALE-deaminase activity values (proportions to AncBE4max activity values) were obtained by the above 7.

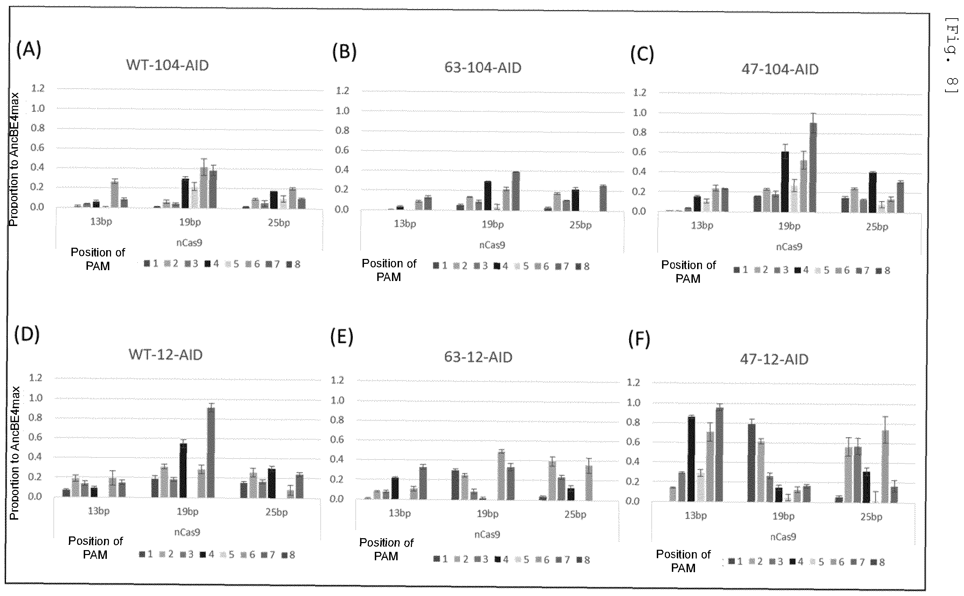

Results are shown in Fig. 8. As shown in Fig. 8, in the TALE-deaminases (AID series) other than 47-12-AID, higher activity was exhibited when the length of the spacer 1 was 19 bp. As the position of the PAM sequence, WT-104-AID exhibited high activity at PAM6 (for example, in the case of the length of the spacer 1: 19 bp, 0.42±0.08) or PAM7 (0.38±0.05) (Fig. 8-A), 63-104-AID at PAM7 (0.39±0.00) (Fig. 8-B), also 47-104-AID at PAM7 (0.9010.10) (Fig. 8-C), also WT-12-AID at PAM7 (0.9110.04) (Fig. 8-D), and 63-12-AID at PAM6 (0.4910.02) (Fig. 8-E) . On the other hand, 47-12-AID exhibited high activity at PAM4 (0.86±0.02) or PAM7 (0.96±0.03) when the length of the spacer 1 was 13 bp, at PAM1 (0.79±0.05) when the length of the spacer 1 was 19 bp, and at PAM6 (0.74±0.13) when the length of the spacer 1 was 25 bp (Fig. 8-F). Among these, 47-104-AID with the length of the spacer 1: 19 bp/PAM7 (0.90±0.10), WT-12-AID with the length of the spacer 1: 19 bp/PAM7 (0.91±0.04), 47-12-AID with the length of the spacer 1: 13 bp/PAM7 (0.96±0.03) exhibited higher activity.

### <Test 4> Effects of the length of the amino acid linker 1 on the activity of the TALE-deaminase

Although it was revealed that activity was higher with 12 amino acids than with 104 amino acids as the amino acid linker 1 from the above Test 3, the amino acids in length were not studied. In view of this, the effects of the length of the amino acid linker 1 was examined by using the TALE-deaminase expression plasmids (47-12' to 84'-AID: the length of the amino acid linker 1: 84 amino acids, 60 amino acids, 36 amino acids, 24 amino acids, and 12 amino acids) prepared in the above 3.(3).

Each TALE-deaminase expression plasmid prepared in the above 3.(3) or a TALE-deaminase-NC expression plasmid corresponding to this, the nCas9 expression plasmid prepared in the above 4., the guide RNA expression plasmid 2 prepared in the above 5., the reporter plasmid (spacer 1: 25 bp) prepared in the above 1., and pGL4.54, which was the reference plasmid, were combined and introduced into the HEK293 T-cells in the above 6., and TALE-deaminase activity values (proportions to AncBE4max activity values) were obtained by the above 7.

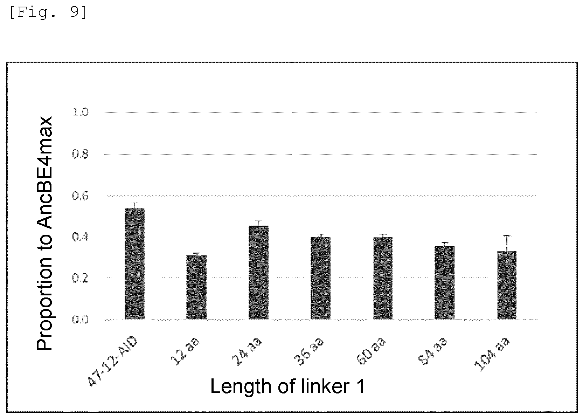

Results are shown in Fig. 9. Fig. 9 also shows results when 47-104-AID prepared in the above 3.(1), or 47-12-AID prepared in the above (2), or a TALE-deaminase-NC expression plasmid corresponding to this was used as the TALE-deaminase expression plasmid together. As shown in Fig. 9, although the activity of the TALE-deaminase (AID) was slightly low in the case where the length of the amino acid linker 1 was shortened to 12 amino acids, it was revealed that the effects of the length of the amino acid linker 1 on the activity were not so much.

### 8. Preparation of TALE-deaminase expression plasmids (2) (1) BE4 series (BE4-TALE)

### (a) 32 Amino acid linker series (BE4-32-TALE)

An expression plasmid of a TALE-deaminase (hereinafter sometimes referred to as "BE4-32-TALE") in which Anc689 deaminase (BE4), a linker 1 (32 amino acids), and TALE (C-terminal domain: WT, 63, or 47) were arranged in this order from the N-terminus was prepared by the following method. Specifically, first, the 5' side (primer SEQ ID NO: 25, 73 to 74) containing a sequence encoding the 32 amino acid linker 1 and the 3' side (primer SEQ ID NO: 12, 75 to 77) containing a sequence encoding a double repeat of the 10 amino acid linker 2 and UGI were amplified by PCR using the primers described in Table 8 given below with the AncBE4max expression plasmid prepared in 3. (1) as a template . In addition, sequences (primer SEQ ID NO: 22 to 24, 78 to 79) encoding the respective TALEs were amplified by PCR with each of the destination vectors ( TALE-WT, TALE-63, and TALE-47) prepared in 2. as a template. The primers used for constructing the BE4-32-TALE expression plasmids and SEQ ID NOs thereof are shown in Table 8 given below.

**[Table 8]**

| Products | Forward Primer (5'-3') | SEQ ID NO | Reverse Primer (5'-3' ) | SEQ ID NO |
|---|---|---|---|---|
| β-lactamase-Anc689 deaminase-32aa Liker for BE4-32-WT, BE4-32-63 | BE4_32aa+infTALEW | 73 | Amp_R | 25 |
| β-lactamase-Anc689 deaminase-32aa Liker for BE4-32-47 | BE4_32aa+infTALE47 | 74 | | |
| 10aa-UGI-10aa-UGI-NLS-β-lactamase for BE4-32-WT | 10aa_F+infWT | 75 | Amp_F | 12 |
| 10aa-UGI-10aa-UGI-NLS-β-lactamase for BE4-32-63 | 10aa_F+inf63 | 76 | | |
| 10aa-UGI-10aa-UGI-NLS-β-lactamase for BE4-32-47 | 10aa_F+inf47 | 77 | | |
| TALE-WT | TALE_WT_F | 78 | TALE_WT_R | 22 |
| TALE-63 | | | TALE_63_R | 23 |
| TALE-47 | TALE_47_F | 79 | TALE_47_R | 24 |

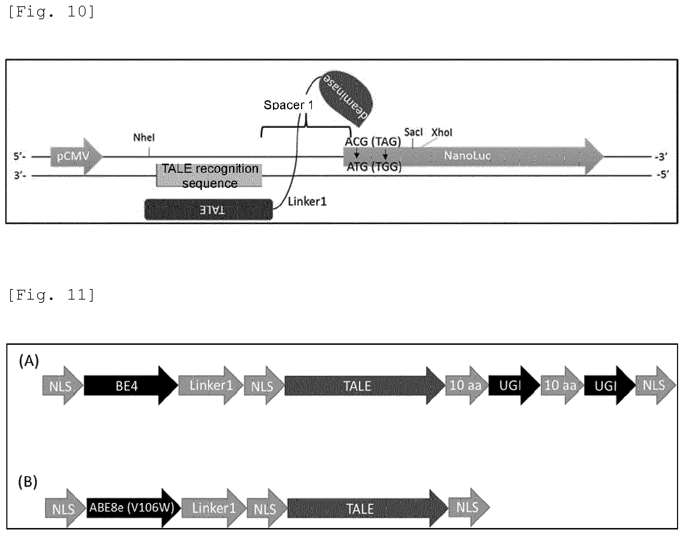

Destination vectors (BE4-32-WT, BE4-32-63, and BE4-32-47) for expressing BE4-32-TALE expression plasmids in which the deaminase (Anc689 deaminase (BE4)), the linker 1 (32 amino acids), the TALE (C-terminal domain: WT, 63, or 47) were arranged in this order from the N-terminus were prepared by the In-Fusion method in the same manner as in 3. (1) by combining the above-described three fragments. Each TALE-deaminase expression plasmid or TALE-deaminase-NC expression plasmid was prepared by the Golden Gate method in the same manner as in 2 . by using these and an array plasmid obtained by linking the TALE sequence prepared in 1., or an array plasmid obtained by linking the NC sequence prepared in 3. A concept diagram showing the structure of the TALE-deaminase is shown in (A) of Fig. 11. In addition, a list of the configurations of the TALE-deaminases and the negative controls (TALE-deaminase-NC) thereof and SEQ ID NOs showing the sequences thereof (SEQ ID NOs of the base sequences and SEQ ID NOs of the amino acid sequences) in the respective expression plasmids thus prepared is shown in Table 12 given below.

### (b) Amino acids in length adjustment linker series (BE4-135/234-TALE)

Subsequently, expression plasmids (the length of the linker 1: 135 or 234 amino acids containing tether sequences added to the front and back of the HTS95 amino acid sequence) of TALE-deaminases (hereinafter, sometimes referred to as "BE4-135-TALE" or "BE4-234-TALE") obtained by inserting a sequence encoding HTS95 amino acid sequence (Sun, N. and Zhao, H., Mol BioSyst 10, p. 446-453 (2014), Doi: 10.1039/c3mb70412b), which was a known linker sequence, or a sequence encoding a double repeat thereof on the downstream side of the sequence encoding the length (32 amino acids) of the linker 1 between deaminases and TALE were prepared by the following method. Specifically, the 5' side (primer SEQ ID NOs: 25, 80) containing a sequence encoding the 32 amino acid linker 1 and the 3' side (primer SEQ ID NOs: 12, 81 to 82) following the sequence encoding each TALE were amplified by PCR using the primers described in Table 9 given below with each of the destination vectors of BE4-32-WT, BE4-32-63, and BE4-32-47 prepared in 8. (1) (a) as a template. The primers used and SEQ ID NOs thereof are shown in Table 9 given below.

**[Table 9]**

| Products | Forward Primer (5'-3') | SEQ ID NO | Reverse Primer (5'-3' ) | SEQ ID NO |
|---|---|---|---|---|
| β-lactamase-Anc689 deaminase-32aa Liker for HTS 95aa linker insertion | BE4_32aa+inf95aa5'-2 | 80 | Amp_R | 25 |
| TALEWT 10aa-UGI-10aa-UGI-NLS β-lactamase for HTS 95aa linker insertion | TALEWT_F+inf95aa3'-2 | 81 | Amp_F | 12 |
| TALE63 10aa-UGI-10aa-UGI-NLS β-lactamase for HTS 95aa linker insertion | | | | |
| TALE47 10aa-UGI-10aa-UGI-NLS β-lactamase for HTS 95aa linker insertion | TALE47_F+inf95aa3'-2 | 82 | | |

In addition, a plasmid containing sequences (HTS95: SEQ ID NO of the base sequence: 105 and the SEQ ID NO of the amino acid sequence: 106) encoding HTS95 amino acid sequence and tether sequences at the front and back thereof was double-digested with restriction enzymes AleI and PshAI, the objective DNA fragment containing a sequence encoding HTS95 was recovered by agarose gel electrophoresis, ligation was conducted by using T4 ligase, followed by double digestion with restriction enzymes XmaI and SacII, and the objective DNA fragment containing HTS95×2 was recovered by agarose gel electrophoresis.

Destination vectors (BE4-135-WT, BE4-135-63, BE4-135-47, BE4-234-WT, BE4-234-63, and BE4-234-47) for expressing BE4-135-TALE or BE4-234-TALE in which the deaminase (Anc689 deaminase (BE4)), the linker 1 (135 amino acids or 234 amino acids), the TALE (C-terminal domain: WT, 63, or 47) were arranged in this order from the N-terminus were prepared by the In-Fusion method in the same manner as in 3. (1) by combining the above-described three fragments. Each TALE-deaminase expression plasmid or TALE-deaminase-NC expression plasmid was prepared by the Golden Gate method in the same manner as in 2. by using these destination vectors and an array plasmid obtained by linking the TALE sequence prepared in 1. , or an array plasmid obtained by linking the NC sequence prepared in 3. A concept diagram showing the structure of the TALE-deaminase is the same as (a) of Fig. 11. In addition, a list of the configurations of the TALE-deaminases and the negative controls (TALE-deaminase-NC) thereof and SEQ ID NOs showing the sequences thereof (SEQ ID NOs of the base sequences and SEQ ID NOs of the amino acid sequences) in the respective expression plasmids thus prepared is shown in Table 12 given below.

### (2) ABE8e series (ABE8e-TALE)

First, ABEmax expression plasmids 2 were prepared by inserting ABEmax gene (Koblan et al., Nat Biotechnol, 36, p. 843-846 (2018)) prepared through artificial DNA synthesis between the BamHI recognition site and the EcoRV recognition site of pcDNA 3.1s.

Subsequently, a plasmid expressing ABE8e(V106W) (Richter, M. F., et al., Nat Biotechnol 38, p. 883-891 (2020), Doi: 10.1038/s41587-020-0453-z), which is an improved version of ABEmax, was prepared. Specifically, a sequence encoding NLS at the N-terminus of the 3'-ABEmax gene to the sequence-5' encoding up to the C-terminus of the 32 amino acid linker 1 were amplified by PCR (primer SEQ ID NOs: 83 to 84) using the primers described in Table 10 given below with the ABEmax expression plasmid 2 as a template to obtain an acceptor. In addition, primers were designed to contain mutations which induced V106W, A109S, T111R, D119N, H122N, Y147D, F149Y, T166I, and D167N on the ABE7.10 gene, and two fragment A (primer SEQ ID NOs : 85 to 86) and fragment B (primer SEQ ID NOs: 87 to 88) were amplified by PCR with the ABEmax expression plasmid 2 as a template. Subsequently, the two fragments were inserted into the acceptors by the In-Fusion method to prepare an ABE8e (TadA-8e(V106W)) expression plasmid. The primers used and SEQ ID NOs thereof are shown in Table 10 given below. Note that in each Table given below, "ABE8e" indicates that the deaminase is TadA-8e(V106W).

**[Table 10]**

| Products | Forward Primer (5'-3') | SEQ ID NO | Reverse Primer (5'-3' ) | SEQ ID NO |
|---|---|---|---|---|
| N-terminal NLS and 32aa Linker-Cas9(D10A)-NLS for ABE8e | 5 "NLS R-infTadA - | 83 | ABE8e-F2 | 84 |
| A fragment for ABE8e | TadA7.1_ F | 85 | ABE8e_R1 | 86 |
| B fragment for ABE8e | ABE8e_F1 | 87 | ABE8e_R2 | 88 |

Subsequently, an expression plasmid of TALE-deaminase (hereinafter, sometimes referred to as "ABE8e-32-47", "ABE8e-135-47", or "ABE8e-234-47") in which the ABE deaminase (ABE8e), the linker 1 (32 amino acids, 135 amino acids, or 234 amino acids), and the TALE (C-terminal domain: 47) were arranged in this order from the N-terminus was prepared by the following method. Specifically, the 5' side containing a sequence encoding NLS at the N terminus and the 3' side containing a sequence encoding from the 32 amino acid linker to the C-terminal side were amplified, containing a vector, by PCR (primer SEQ ID NO: 89 to 90) using primers described in Table 11 given below with each of the destination vectors BE4-32-47, BE4-135-47, or BE4-234-47 prepared in 8. (1) (a) and (b) as a template to obtain an acceptor. In addition, a ABE8e(TadA-8e(V106W)) gene portion amplified by PCR (addition of primer SEQ ID NOs: 91 to 92) with the ABE8e(TadA-8e(V106W)) expression plasmid as a template was inserted into the acceptor by the In-Fusion method to prepare a destination vector for expressing ABE8e-32-47, ABE8e-135-47, or ABE8e-234-47. The primers used and SEQ ID NOs thereof are shown in Table 11 given below.

**[Table 11]**

| Products | Forward Primer (5'-3') | SEQ ID NO | Reverse Primer (5'-3' ) | SEQ ID NO |
|---|---|---|---|---|
| N-terminal NLS and 32aa Linker-TALE-NLS for ABE8e-TALE | SV40NLS_R | 89 | 32aaLinker_F | 90 |
| ABE8e-V82G for ABE8e-TALE | ABE8e_Rinf32aa | 91 | ABE8e_FinfNLS | 92 |

Each TALE-deaminase expression plasmid or TALE-deaminase-NC expression plasmid was prepared by the Golden Gate method in the same manner as in 2. by using each destination vector and an array plasmid obtained by linking the TALE sequence prepared in 1., or an array plasmid obtained by linking the NC sequence prepared in 3. A concept diagram showing the structure of the TALE-deaminase is shown in (B) of Fig. 11. In addition, a list of the configurations of the TALE-deaminases and the negative controls (TALE-deaminase-NC) thereof and SEQ ID NOs showing the sequences thereof (SEQ ID NOs of the base sequences and SEQ ID NOs of the amino acid sequences) in the respective expression plasmids thus prepared is shown in Table 12 given below.

**[Table 12]**

| TALE-deaminase | | | | | | TALE-deaminase-NC (negative control) | | | | | |
|---|---|---|---|---|---|---|---|---|---|---|---|
| ID | TALE recognitio n sequence | TALE C-termina l domain | Length of linker 1 (a.a.) | Deaminas e | SEQ ID NO | ID | TALE recognitio n sequence | TALE C-terminal domain | Length of linker 1 (a.a.) | Deaminase | SEQ ID NO |
| BE4-32 -WT | | WT | 32 | | 93, 94 | BE4-32 -WT-NC | | WT | 32 | | 95, 96 |
| BE4-13 5-WT | | | 135 | | 107 108 | BE4-13 5-WT-N C | | | 135 | | 109 110 |
| BE4-23 4-WT | | | 234 | | 119 120 | BE4-23 4-WT-N C | | | 234 | | 121 122 |
| BE4-32 -63 | | 63 | 32 | | 97, 98 | BE4-32 -63-NC | | 63 | 32 | | 99, 100 |
| BE4-13 5-63 | | | 135 | Anc689 | 111 112 | BE4-13 5-63-N C | | | 135 | Anc689 | 113 114 |
| BE4-23 4-63 | | | 234 | | 123 124 | BE4-23 4-63-N C | | | 234 | | 125 126 |
| BE4-32 -47 | human | 47 | 32 | | 101 102 | BE4-32 -47-NC | Non-human | 47 | 32 | | 103 104 |
| BE4-13 5-47 | | | 135 | | 115 116 | BE4-13 5-47-N C | | | 135 | | 117 118 |
| BE4-23 4-47 | | | 234 | | 127 128 | BE4-23 4-47-N C | | | 234 | | 129 , 130 |
| ABE8e-32-47 | | 47 | 32 | TadA-8e (V106W) | 131 , 132 | ABE8e-32-47-NC | | 47 | 32 | TadA-8e (V106W) | 133 , 134 |
| ABE8e-135-47 | | | 135 | | 135 136 | ABE8e-135-47 -NC | | | 135 | | 137 138 |
| ABE8e-234-47 | | | 234 | | 139 140 | ABE8e-234-47 -NC | | | 234 | | 141 142 |

### 9. Preparation of TALE-deaminase expression plasmids targeting endogenous DNA (genomic DNA)

As TALE recognition sequences, array plasmids obtained by linking TALE sequences in the same method as in 2. were prepared to target eight types of sequences on endogenous DNAs described in Table 13 given below. As the endogenous DNAs, CCR5 and HBB for which family genes with high homology (CCR2 and HBD) exist were set.

Each expression plasmid (TALE-AID expression plasmid) of a TALE-deaminase (WT(*)-12-AID, 47(*)-12-AID, 47(*)-104-AID: * each represent a target) in which the TALE (C-terminal domain: WT or 47), the linker 1 (12 amino acids or 104 amino acids), and the deaminase (AID) were arranged in this order from the N-terminus was prepared by the Golden Gate method in the same manner as in 3. except that each of the above-described array plasmids was used. In addition, each expression plasmid (BE4-TALE expression plasmid, ABE8e-TALE expression plasmid) of a TALE-deaminase (BE4-32-47(*), BE4-135-63(*), ABE8e-135-47(*): * each represent a target) in which the deaminase (BE4 or ABE8e), the linker 1 (32 amino acids or 135 amino acids), and the TALE (C-terminal domain: 63 or 47) were arranged in this order from the N-terminus by the Golden Gate method in the same manner as in 8. except that each array plasmid was used. The targeted TALE recognition sequences are shown in Table 13, and a list of the targets, the configurations of the TALE-deaminases, and SEQ ID NOs showing the sequences thereof (SEQ ID NOs of the base sequences and SEQ ID NOs of the amino acid sequence) in the respective expression plasmids prepared is shown in Table 14.

**[Table 13]**

| TALE | TALE recognition sequence (5'-3') | SEQ ID NO |
|---|---|---|
| TALE-AS3 | tGAGCCCAGAAGGGGACA | 143 |
| TALE-AS14-1 | tAGCTTGGtCCAACCTGT | 144 |
| TALE-AS14-2 | tGGTCAACCTGTTAGAGC | 145 |
| TALE-AS14-3 | tCTAACAGGTTGGACCAA | 146 |
| TALE-AS5 | tGCAGTAGCTCTAACAGG | 147 |
| TALE-AS 6 | tCTGCCGTTACTGCCCTG | 148 |
| TALE-AS7 | tTTGCCACACTGAGTGAG | 149 |
| TALE-S2 | tGCCACACTGAGTGAGC | 150 |

**[Table 14]**

| ID | Target | Name of TALE | TALE C-terminal domain | Length of linker 1 (a.a.) | Deaminase | SEQ ID NO |
|---|---|---|---|---|---|---|
| WT (AS3)-12-AID | AS3 | TALE-AS3 | WT | 12 | PmCDA1 | 151, 152 |
| 47(AS3)-104-AID | | | 47 | 104 | | 153, 154 |
| WT (AS14-1)-12-AID | AS14 | TALE-AS14-1 | WT | 12 | | 155, 156 |
| 47 (AS14-1)-104-AID | | | 47 | 104 | | 157, 158 |
| 47 (AS14-2)-12-AID | | TALE-AS14-2 | 47 | 12 | | 159, 160 |
| WT (S2)-12-AID | S2 | TALE-S2 | WT | 12 | | 161, 162 |
| 47 (S2)-104-AID | | | 47 | 104 | | 163, 164 |
| BE4-32-47 (AS14-3) | AS14 | TALE-AS14-3 | 47 | 32 | Anc689 | 165, 166 |
| BE4-135-63 (AS14-3) | | | 63 | 135 | | 167, 168 |
| BE4-32-47 (AS5) | ASS | TALE-AS5 | 47 | 32 | | 169, 170 |
| BE4-135-63 (AS5) | | | 63 | 135 | | 171, 172 |
| BE4-32-47(AS6) | AS6 | TALE-AS6 | 47 | 32 | | 173, 174 |
| BE4-135-63 (AS6) | | | 63 | 135 | | 175, 176 |
| BE4-32-47 (AS7) | AS7 | TALE-AS7 | 47 | 32 | | 177, 178 |
| BE4-135-63 (AS7) | | | 63 | 135 | | 179, 180 |
| ABE8e-135-47 (AS5) | AS5 | TALE-AS5 | 47 | 135 | TadA-8e (V106W) | 181, 182 |
| ABE8e-135-47 (AS6) | AS6 | TALE-AS6 | | | | 183, 134 |
| ABE8e-135-47 (AS7) | AS7 | TALE-AS7 | | | | 185, 186 |

### 10. Preparation of reporter plasmids for BE4-TALE and ABE8e-TALE

As reporters, reporter plasmids for BE4-TALE and ABE8e-TALE were prepared by inserting sequences (N-CBE (for BE4-TALE) or N-ABE (for ABE8e-TALE)) shown in Table 15 given below into pNLF-M/A prepared in 1. The insertion sequence contained a complementary sequence of the TALE recognition sequence (the underline(*1): 5'-CCTTTGCCCGCTTCTGTa-3' in Table 15; the lower-case letter indicates a complementary base of thymine recognized by the N-terminal domain of TALE), a target codon (the underline(*2): 5'-ACG-3' or 5'-TAG-3' in Table 15), and a spacer 1 sequence therebetween, and the length of the spacer 1 was set to 7 bp, 13 bp, 19 bp, 25 bp, or 31 bp containing the base at 5' of the target codon. The sequences inserted in the pNLF-M/A plasmid are shown in Table 15 given below.

**[Table 15]**

| Products | Sequence (5'-3') | SEQ ID NO |
|---|---|---|
| N-CBE-7bp | CTAGCATAAAACCTTTGCCCGCTTCTGTa^{*1}GGGAAGACG^{*2}GATATCTCCTGCGGGG^{*3}GAGCT | 187 |
| N-CBE-13bp | CTAGCATAAAACCTTTGCCCGCTTCTGTa^{*1}CTATTTGGGAAGACG^{*2}GATATCTCCTGCGGGG^{*3}GAGCT | 188 |
| N-CBE-19bp | CTAGCATAAAACCTTTGCCCGCTTCTGTa^{*1}ATTAGTCTATTTGGGAAGACG^{*2}GATATCTCCTGCGGGG^{*3}GAGCT | 189 |
| N-CBE-25bp | CTAGCATAAAACCTTTGCCCGCTTCTGTa^{*1}TTCAGGATTAGTCTATTTGGGAAGACG^{*2}GATATCTCCTGCGGGG^{*3}GAGCT | 190 |
| N-CBE-31bp | CTAGCATAAAACCTTTGCCCGCTTCTGTa^{*1}AGTCTATTCAGGATTAGTCTATTTGGGAAGACG^{*2}GATATCTCCTGCGGGG^{*3}GAGCT | 191 |
| N-ABE-7bp | CTAGCACCATGGCGCCTTTGCCCGCTTCTGTa^{*1}CGCGCTTAG^{*2}TCTTTCGGC | 192 |
| N-ABE-13bp | CTAGCACCATGGCGCCTTTGCCCGCTTCTGTa^{*1}GCCTCCCGCGCTTAG^{*2}TCTTTCGGC | 193 |
| N-ABE-19bp | CTAGCACCATGGCGCCTTTGCCCGCTTCTGTa^{*1}CCCAACGCCTCCCGCGCTTAG^{*2}TCTTTCGGC | 194 |
| N-ABE-25bp | CTAGCACCATGGCGCCTTTGCCCGCTTCTGTa^{*1}GCAGGCCCCAACGCCTCCCGCGCTTAG^{*2}TCTTTCGGC | 195 |
| N-ABE-31bp | CTAGCACCATGGCGCCTTTGCCCGCTTCTGTa^{*1}GCTGGAGCAGGCCCCAACGCCTCCCGCGCTTAG^{*2}TCTTTCGGC | 196 |

| | | |
|---|---|---|
| *1: Complementary sequence of TALE recognition sequence *2: Target codon containing target base *3: PAM | | |

In this reporter plasmid for BE4-TALE, the target codon was located at a position corresponding to the start codon of Nano luciferase, and this reporter plasmid expresses NanoLuc luciferase when ACG, which is the target codon, is converted to ATG (C, which is the target base (target site), is substituted with T) by the TALE-deaminase bound to the TALE recognition sequence (Fig. 10) . On the other hand, in the reporter plasmid for ABE8e-TALE, the target codon is located at a position corresponding to the termination codon inserted upstream of NanoLuc luciferase, and this reporter plasmid expresses NanoLuc luciferase when TAG, which is the target codon, is converted to TGG (A, which is the target base (target site), is substituted with G) by the TALE-deaminase bound to the TALE recognition sequence.

### 11. Preparation of guide RNA expression plasmids (2)

A Plasmid (guide RNA expression plasmid) expressing each guide RNAs was prepared in the same manner as in 5. except that the plasmid expressing the guide RNA was designed to correspond to the target sequence (complementary sequences of the target sequences (on the antisense strand) of the guide RNAs are shown in Table 16) of the guide RNA. The complementary sequences of the target sequences of the guide RNAs are shown in Table 16 given below.

**[Table 16]**

| Guide RNA | Target | Complementary sequence of target sequence of guide RNA | SEQ ID NO |
|---|---|---|---|
| sgRNA-N-CBE | Reporter for BE4-TALE | GAAGACGGATATCTCCTGCG | 197 |
| sgRNA-N-ABE | Reporter for ABE8e-TALE | GCTTAGTCTTTCGGCTCGAG | 198 |
| sgRNA-AS3 | AS3 | CAGAGATGGCCAGGTTGAGC | 199 |
| sgRNA-AS14 | AS14 | CAGGCCAAAGAATTCCTGGA | 200 |
| sgRNA-AS5 | AS5 | AGAATTCCTGGAAGGTGTTC | 201 |
| sgRNA-AS6 | AS 6 | TGCACCATGGTGTCTGTTTG | 202 |
| sgRNA-AS7 | AS7 | GTGAGCCAGGCCATCACTAA | 203 |
| sgRNA-S2 | S2 | CGTGGATCCTGAGAACTTCA | 204 |

### 12. Transformation to HEK cells (2)

In the case where each of the reporter plasmids prepared in the above 10. was used as the target DNA, the same method as shown in 6. was conducted. Specifically, into 5×10⁴ HEK293 T-cells in each well, 75 ng of one of the BE4-TALE expression plasmids and the ABE8e-TALE expression plasmids prepared in 8.; 50 ng of the nCas9 expression plasmid or dCas9 expression plasmid prepared in 4.; 10 ng of the guide RNA expression plasmid prepared in 11. or 20 ng of one of the reporter plasmids prepared in 10. ; and 5 ng of pGL4.54, which is the reference plasmid, were introduced by using Lipofectamine LTX, followed by culturing for 24 hours.

In addition, in the case where the endogenous DNA described in the above 9. was used as the target DNA, the following was conducted. Specifically, a combination of 30 ng of one of the TALE-AID expression plasmids prepared in 9.; 20 ng of the nCas9 expression plasmid or the dCas9 expression plasmid prepare in 4.; and 5 ng of the guide RNA expression plasmid prepared in 11. in the case where AID was used as the deaminase, and a combination of 60 ng of one of the BE4-TALE expression plasmid or the ABE8e-TALE expression plasmid prepared in 9. ; 40 ng of the nCas9 expression plasmid or dCas9 expression plasmid prepared in 4.; and 10 ng of the guide RNA expression plasmid prepared in 11. in the case where BE4 or ABE8e was used as the deaminase, were each introduced into 3×10⁴ HEK293 T-cells per well, followed by culturing for 48 hours. In addition, as the control in this case, one of a combination of 30 ng of one of the Target-AID expression plasmid, the AncBE4max expression plasmid, and the ABE8e expression plasmid and 10 ng of the guide RNA expression plasmid prepared in 11., or a combination of 60 ng of one of the Target-AID expression plasmid, the AncBE4max expression plasmid, and the ABE8e expression plasmid and 20 ng of the guide RNA expression plasmid prepared in 11. was introduced into the HEK293 T-cells.

### 13. Analysis of the base editing activity

The medium was removed 48 hours after the transformation, followed by washing with PBS (-), and thereafter, 50 µL of DNAzol (Molecular Research Center, INC., Ohio, USA) was added to dissolve the cells. The region containing the target site of the endogenous DNA was amplified by using PrimeSTAR Max (Takara Bio Inc., Siga, Japan) using the primers described in Table 17 given below with cell lysates as a template. After the PCR product thus obtained was purified, the sequence was outsourced to FASMAC. Sequence data were analyzed by using EditR (Kluesner et al., The CRISPR J 1, p. 239-250 (2018), DOI: 10.1089/crispr.2018.0014) to calculate the base editing efficiency (proportion of T or G) of the target site.

**[Table 17]**

| Target | Target gene | Forward Primer (5'-3') | SEQ ID NO | Reverse Primer (5'-3' ) | SEQ ID NO |
|---|---|---|---|---|---|
| AS3 | CCR5 | CCR5-AS3-F2 | 205 | CCR5-AS3-R2 | 206 |
| | CCR2 | CCR2-AS3-F | 207 | CCR2-AS3-R | 208 |
| AS14/AS5 | CCR5 | CCR5-AS14-F1 | 209 | CCR5-AS14-R | 210 |
| | CCR2 | CCR2-AS14-F1 | 211 | CCR2-AS14-R1 | 212 |
| AS6 | HBB | HBB-AS6-F2 | 213 | HBB-AS6-R2 | 214 |
| | HBD | HBD-AS6-F | 215 | HBD-AS6-R | 216 |
| AS7/S2 | HBB | HBB-S2-F | 217 | HBB-S2-R | 218 |
| | HBD | HBD-S2-F | 219 | HBD-S2-R | 220 |

### <Test 5> Effects of the length of the spacer 1 and the length of the linker 1 on the activity of the TALE-deaminase

### (N-terminal fusion product)

Transformation to HEK cells was conducted by the above 12., and TALE-deaminase activity values (proportions to the AncBE4max activity values or the ABE8e activity values) were obtained by the above 7.

### (BE4 series)

For the TALE-deaminase (BE4-TALE-WT, BE4-TALE-63, or BE4-TALE-47) in which the Anc689 deaminase (BE4), the linker 1 (32 amino acids, 135 amino acids, or 234 amino acids), and the TALE (C-terminal domain: WT, 63, or 47) were arranged in this order from the N-terminus, the effects of the length of the spacer 1 and the length of the linker 1 of the reporter on the activity were examined. The length of the spacer 1 was set to 7 bp, 13 bp, 19 bp, 25 bp, and 31 bp. In addition, as Cas9/guide RNA, three conditions where nCas9 (D10A) and the guide RNA were expressed (nCas9), where dCas9 (D10A+H840A) and the guide RNA were expressed (dCas9), where nCas9 was expressed and the guide RNA was not expressed (nCas9/no guide) were studied.

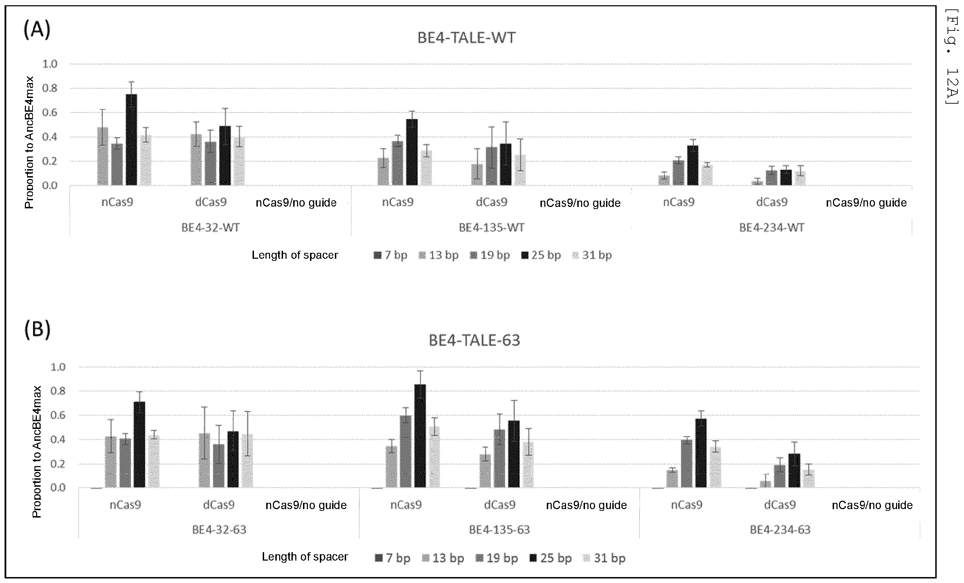

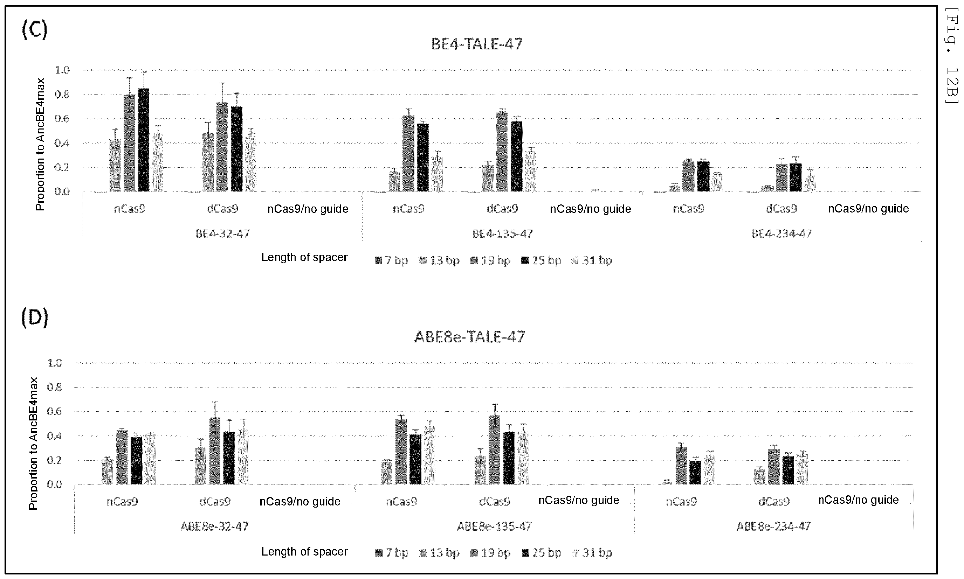

Results are shown in (A) and (B) of Fig. 12A and (C) of Fig. 12B. In any of BE4-TALE, in the case where there was no guide RNA, that is, in the case where nCas9 was not bound in the vicinity of the target base, no activity was observed. On the other hand, the activity was observed in the presence of nCas9 or dCas9. The effects to promote the activity of nicks by nCas9 were not as significant as the C-terminal fusion product of TALE-deaminase (the deaminase was arranged on the C-terminal side).

Regarding the length of the spacer 1, particularly high activity was observed when the length was 25 bp in the case where the C-terminal domain of the TALE was WT and 63, and when the length was 19 bp and 25 bp in the case where the C-terminal domain of the TALE was 47. Regarding the length of the linker 1, particularly high activity was observed when the length was 32 amino acids in the case where the C-terminal domain of the TALE was WT and 47, and when the length was 135 amino acids in the case where the C-terminal domain of the TALE was 63. Combinations with particularly high activities were the case where the length of the linker 1 was 32 amino acids and the length of the spacer 1 was 25 bp in the case where the C-terminal domain of the TALE was WT (0.7510.10), the case where the length of the linker 1 was 135 amino acids and the length of the spacer 1 was 25 bp in the case where the C-terminal domain of the TALE was 63 (0.8610.11), and the case where the length of the linker 1 was 32 amino acids and the length of the spacer 1 was 19 bp and 25 bp in the case where the C-terminal domain of the TALE was 47 (0.80±0.14 and 0.85±0.13).

### (ABE8e series)

For the TALE-deaminase (ABE8e-TALE-47) in which the ABE8e deaminase (ABE8e), the linker 1 (32 amino acids, 135 amino acids, or 234 amino acids), and the TALE (C-terminal domain: 47) were arranged in this order from the N-terminus as well, the effects of the length of the spacer 1 and the length of the linker 1 of the reporter on the activity were examined in the same manner as for the above BE4 series.

Results are shown in (D) of Fig. 12B. In any of ABE8e-TALE, in the case where there was no guide RNA, that is, in the case where nCas9 was not bound in the vicinity of the target base, no activity was observed. On the other hand, the activity was observed in the presence of nCas9 or dCas9. Note that in comparison between nCas9 and dCas9, the activity was at a similar level, or rather higher in the case of dCas9.

Regarding the length of the spacer 1, particularly high activity was observed when the length was 19 bp. Regarding the length of the linker 1, particularly high activity was observed when the length was 135 amino acids (0.5410.03) in the case of nCas9, and when the length was 32 amino acids (0.5510.13) and 135 amino acids (0.57±0.09) in the case of dCas9.

### <Test 6> The base editing activity in endogenous DNA

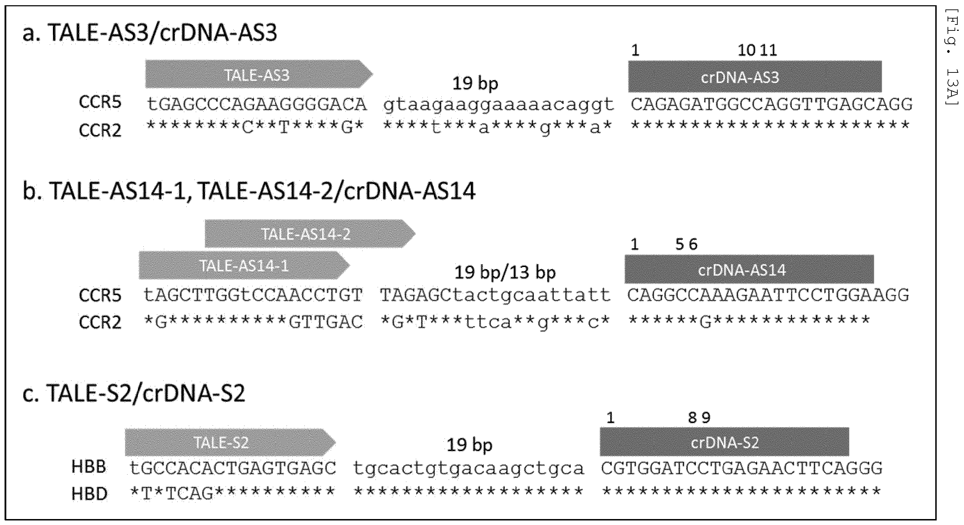

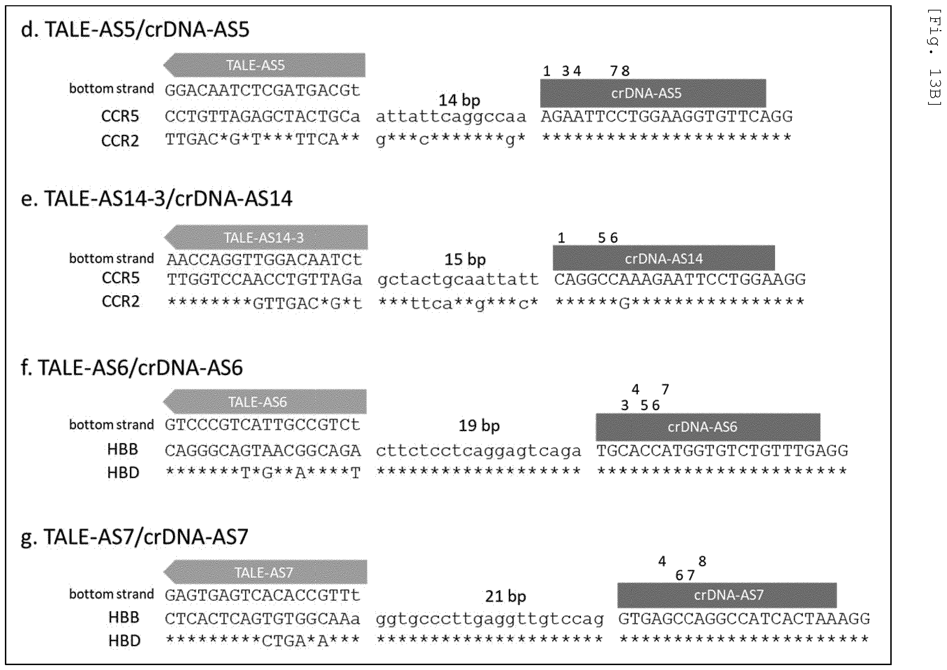

For the TALE-deaminases with which particularly high activity was obtained in the above examination, the base editing activity on the endogenous DNAs was examined by the methods described in the above 12. and 13. As the endogenous DNAs, CCR5 and HBB for which family genes with high homology (CCR2 and HBD) exist were set. Target regions (AS3 (a: SEQ ID NO: 221), AS14 (b: SEQ ID NO: 222) (e: SEQ ID NO: 225), S2 (c: SEQ ID NO: 223), AS5 (d: SEQ ID NO: 224), AS6 (f: SEQ ID NO: 226), AS7 (g: SEQ ID NO: 227)) on the endogenous DNAs (CCR5, HBB), the positions of the TALE recognition sequences or complementary sequences thereof, the positions of the complementary sequences of the target sequences of the guide RNA are shown in Fig. 13A and Fig. 13B. In addition, in Fig. 13A and Fig. 13B, bases different from the bases in each region are also shown as alignments of CCR2 having high homology with CCR5 shown in (a), (b), (d), and (e) and HBD having high homology with HBB shown in (c), (f), and (g).

### (AID series)

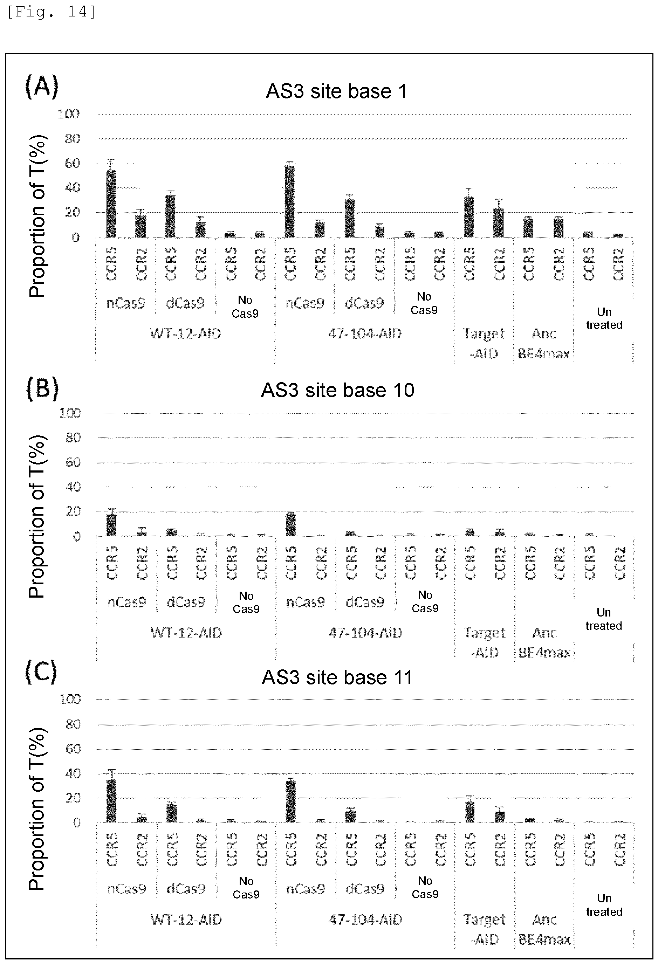

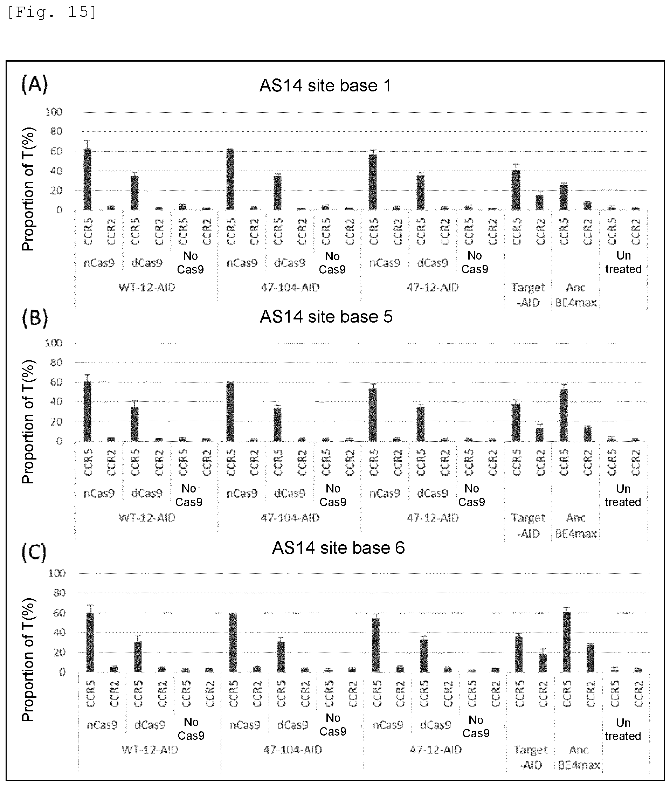

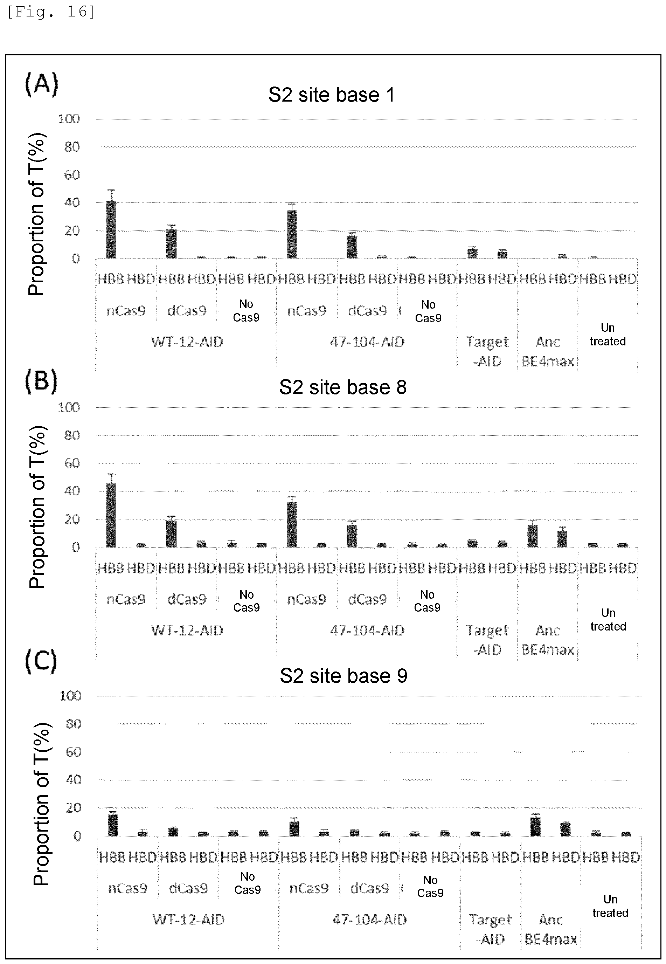

Results of using TALE-AIDs (WT(*)-12-AID, 47(*)-12-AID, and 47(*)-104-AID: * each represents the target) in which the TALE (C-terminal domain: WT or 47), the linker 1 (12 amino acids or 104 amino acids), and the deaminase (AID) were arranged in this order from the N-terminus are shown in Fig. 14 to Fig. 16. In the respective TALE-AIDs, the TALE recognition sequences are located on AS3 (a), AS14 (b), and S2 (c) shown in Fig. 13A, respectively. Fig. 14 shows the proportions of T (that is, the proportions of bases edited from C to T) at the 1st base (A), the 10th base (B), and the 11th base (C) in the complementary sequences of the target sequences of the guide RNA (sgRNA-AS3) on the target AS3, Fig. 15 shows the proportions of T at the 1st base (A), the 5th base (B), and the 6th base (C) in the complementary sequences of the target sequences of the guide RNA (sgRNA-AS14) on the target AS14, and Fig. 16 shows the proportions of T at the 1st base (A), the 8th base (B), and the 9th base (C) in the complementary sequences of the target sequences of the guide RNA (sgRNA-S2) on the target S2.

TALE-AID exhibited activity at a level similar to or higher than those of Target-AID and AncBE4max, which are existing Base editors, for the CCR5 gene or the HBB gene, which was the target. On the other hand, although Target-AID and AncBE4max exhibited base editing activity for the CCR2 gene or the HBD gene, which was not the target, TALE-AID exhibited almost no base editing activity. Hence, it was confirmed that TALE-AID had higher target specificity than those of the existing Target-AID or AncBE4max.

### (BE4 series)

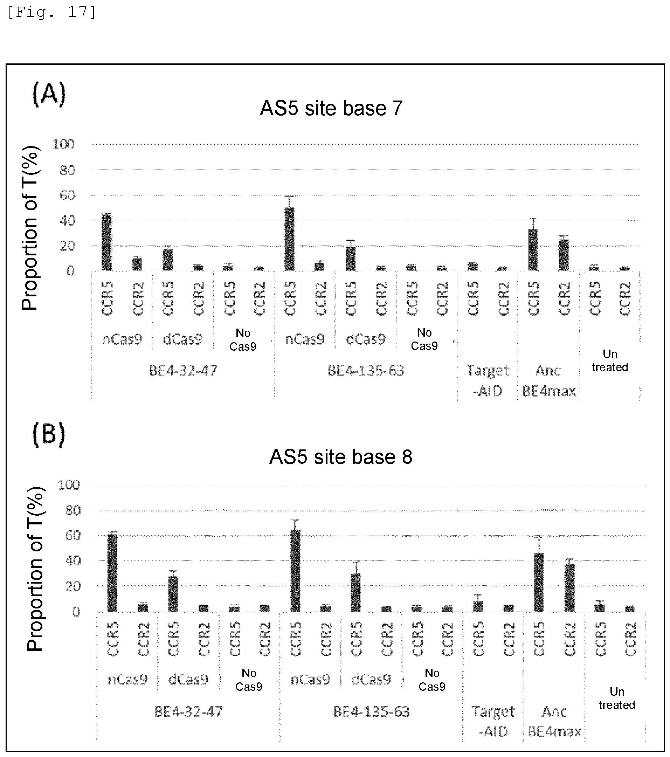

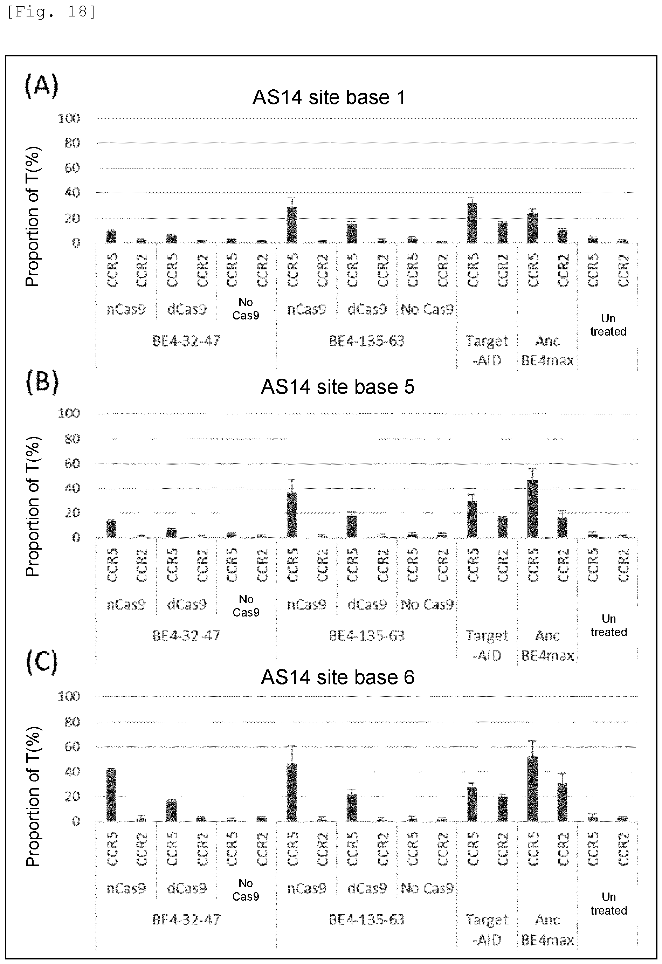

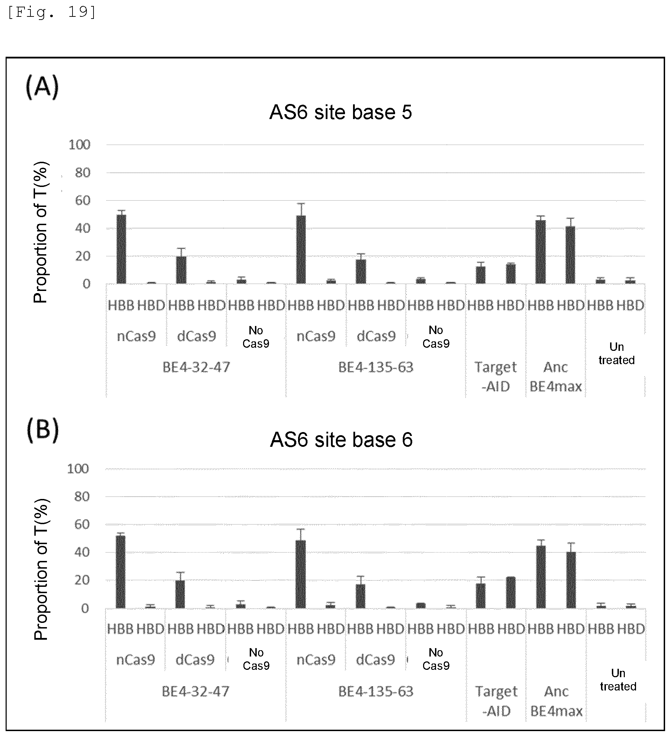

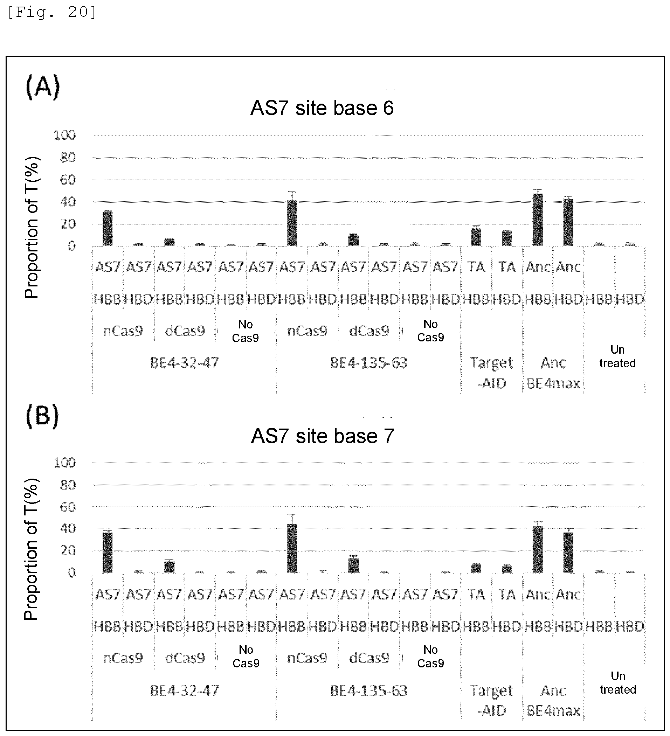

Results of using BE4-TALEs (BE4-32-47(*) and BE4-135-63(*): * each represents the target) in which the deaminase (BE4), the linker 1 (32 amino acids or 135 amino acids), and the TALE (C-terminal domain: 47 or 63) were arranged from the N-terminus are shown in Fig. 17 to Fig. 20. In each BE4-TALE, the TALE recognition sequences were located on complementary sequences of AS5 (d), AS14 (e), AS6 (f), and AS7 (g) shown in Fig. 13B. Fig. 17 shows the proportions of T at the 7th base (A) and the 8th base (B) in the complementary sequences of the target sequences of the guide RNA (sgRNA-AS5) on the target AS5, Fig. 18 shows the proportions of T at the 1st base (A), the 5th base (B), and the 6th base (C) in the complementary sequences of the target sequences of the guide RNA (sgRNA-AS14) on the target AS14, Fig. 19 shows the proportions of T at the 5th base (A) and the 6th base (B) in the complementary sequences of the target sequences of the guide RNA (sgRNA-AS6) on the target AS6, and Fig. 20 shows the proportions of T at the 6th base (A) and the 7th base (B) in the complementary sequences of the target sequences of the guide RNA (sgRNA-AS7) on the target AS7.

BE4-TALE exhibited activity at a level substantially similar to those of Target-AID and AncBE4max, which are existing Base editors, for the CCR5 gene or the HBB gene, which was the target. On the other hand, although Target-AID and AncBE4max exhibited base editing activity for the CCR2 gene or HBD gene, which was not the target, BE4-TALE exhibited almost no base editing activity. Hence, it was confirmed that BE4-TALE had higher target specificity than those of the existing Target-AID or AncBE4max.

### (ABE8e series)

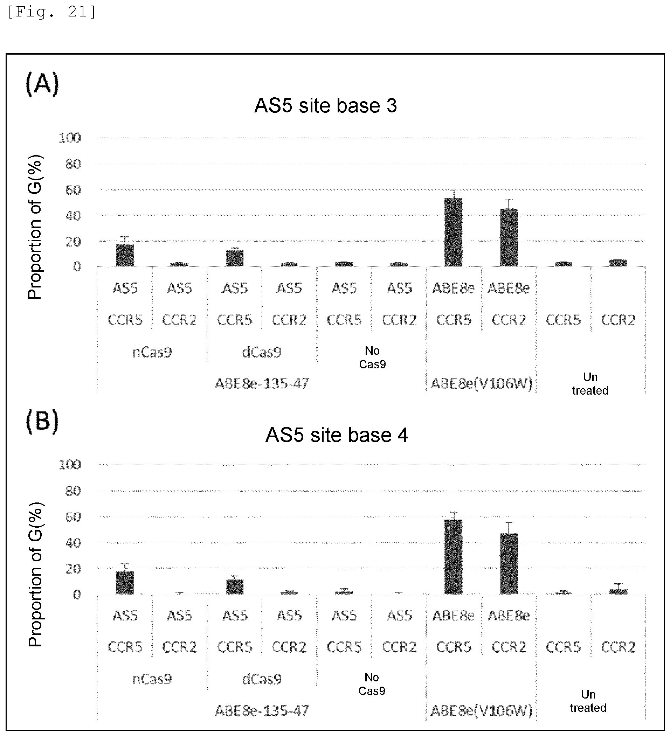

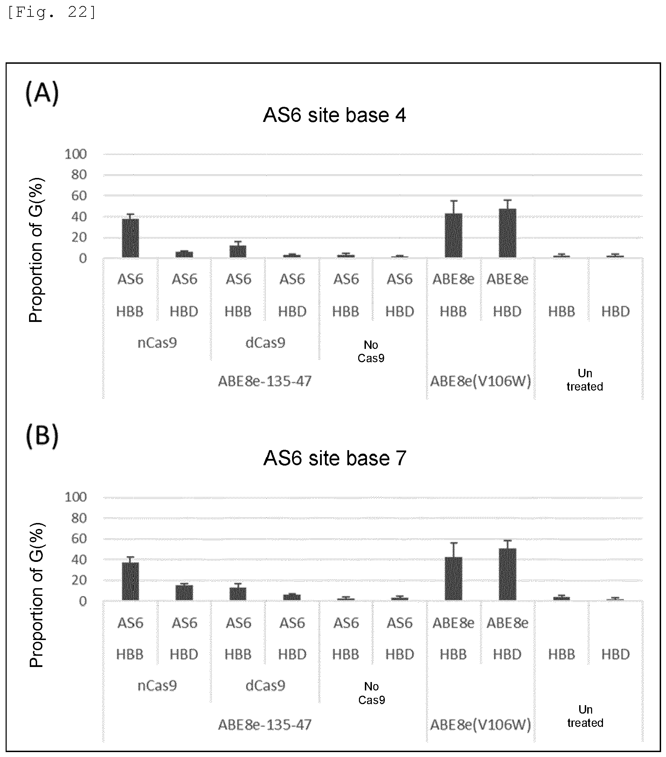

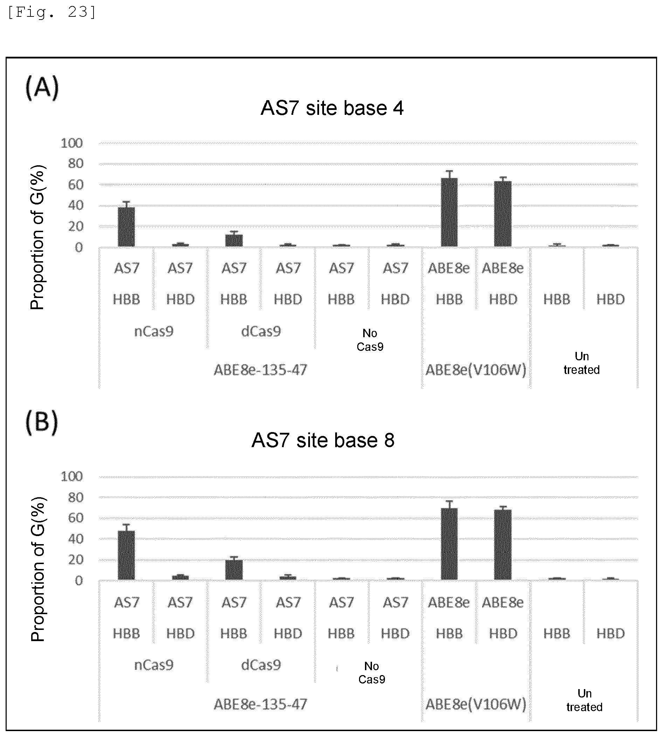

Results of using ABE8e-TALE (ABE8e-135-47) in which the ABE8e deaminase (ABE8e), the linker 1 (135 amino acids), and the TALE (C-terminal domain: 47) were arranged from the N-terminus are shown in Fig. 21 to Fig. 23. In each ABE8e-TALE, the TALE recognition sequences were located on complementary sequences of AS5 (d), AS6 (f), and AS7 (g) shown in Fig. 13B. Fig. 21 shows the proportions of G (that is, the proportions of bases edited from A to G) at the 3rd base (A) and the 4th base (B) in the complementary sequences of the target sequences of the guide RNA (sgRNA-AS5) on the target AS5, Fig. 22 shows the proportions of G at the 4th base (A) and the 7th base (B) in the complementary sequences of the target sequences of the guide RNA (sgRNA-AS6) on the target AS6, and Fig. 23 shows the proportions of G at the 4th base (A) and the 8th base (B) in the complementary sequences of the target sequences of the guide RNA (sgRNA-AS7) on the target AS7.

ABE8e-TALE exhibited activity similar to that of ABE8e (TadA-8e(V106W)), which is an existing Base editor, for the CCR5 gene or the HBB gene, which was the target. On the other hand, although ABE8e (TadA-8e(V106W)) exhibited base editing activity for the CCR2 gene or the HBD gene, which was not the target, ABE8e-TALE exhibited almost no base editing activity. Hence, it was confirmed that ABE8e-TALE had higher target specificity than that of the existing ABE8e (TadA-8e(V106W)).

### [Industrial Applicability]

As described above, the present invention makes it possible to provide a method capable of specifically and efficiently editing a target DNA by using a nucleic acid base converting enzyme, a method for producing a cell genome-edited by using the method, and a DNA editing system for use in these. Hence, the present invention is expected to be utilized in the fields of gene-editing treatment and the like where high editing efficiency and high safety are required.

[Sequence Listing] IBPF21-536WO(seq)-fin.txt

## Claims

1. A method for editing a target DNA comprising:
a step of bringing
(1) a fusion protein containing a TALE and a nucleic acid base converting enzyme and
(2) a CRISPR-Cas9 system containing a Cas9 protein which has lost part or all of a nuclease activity and a guide RNA thereof into contact with a target DNA to edit a base in a target site of the target DNA by using a nucleic acid base converting enzyme activity of the fusion protein, wherein
a TALE recognition sequence recognized by the TALE in the fusion protein or a complementary sequence thereof is present on a 5' side of the target site via a spacer 1 having a chain length of 7 to 31 bp, and
a guide RNA-target sequence recognized by the guide RNA in the CRISPR-Cas9 system is present to contain a complementary base of the target site.

2. A method for producing a cell in which a target DNA is edited, comprising:
a step of introducing or expressing
(1) a fusion protein containing a TALE and a nucleic acid base converting enzyme and
(2) a CRISPR-Cas9 system containing a Cas9 protein which has lost part or all of a nuclease activity and a guide RNA thereof
into a cell or in a cell to bring the fusion protein and the CRISPR-Cas9 system into contact with a target DNA to edit a base in a target site of the target DNA by using a nucleic acid base converting enzyme activity of the fusion protein, wherein
a TALE recognition sequence recognized by the TALE in the fusion protein or a complementary sequence thereof is present on a 5' side of the target site via a spacer 1 having a chain length of 7 to 31 bp, and
a guide RNA-target sequence recognized by the guide RNA in the CRISPR-Cas9 system is present to contain a complementary base of the target site.

3. The method according to claim 1 or 2, wherein
the fusion protein further contains a linker 1 which binds the TALE and the nucleic acid base converting enzyme.

4. The method according to any one of claims 1 to 3, wherein
the fusion protein further contains a base excision repair inhibitor which is bound to a C-terminal side via a linker 2.

5. The method according to any one of claims 1 to 4, wherein
the nucleic acid base converting enzyme in the fusion protein is a deaminase.

6. The method according to claim 5, wherein
the deaminase is at least one selected from the group consisting of APOBEC, PmCDA1, Anc689, and TadA.

7. A DNA editing system for use in the method according to any one of claims 1 to 6, comprising:
(1) a fusion protein containing a TALE and a nucleic acid base converting enzyme; and
(2) a CRISPR-Cas9 system containing a Cas9 protein which has lost part or all of a nuclease activity and a guide RNA thereof.

8. The DNA editing system according to claim 7, wherein
the fusion protein further contains a linker 1 which binds the TALE and the nucleic acid base converting enzyme.

9. The DNA editing system according to claim 7 or 8, wherein
the fusion protein further contains a base excision repair inhibitor which is bound to a C-terminal side via a linker 2.

10. The method according to any one of claims 7 to 9, wherein
the nucleic acid base converting enzyme in the fusion protein is a deaminase.

11. The DNA editing system according to claim 10, wherein
the deaminase is at least one selected from the group consisting of APOBEC, PmCDA1, Anc689, and TadA.
